(19) Europäisches Patentamt
European Patent Office
Office européen des brevets

(11) EP 1 652 922 A1

(12) **EUROPEAN PATENT APPLICATION**
published in accordance with Art. 158(3) EPC

(43) Date of publication:
**03.05.2006 Bulletin 2006/18**

(21) Application number: **04771560.2**

(22) Date of filing: **05.08.2004**

(51) Int Cl.:
*C12N 15/11* (1990.01) *C12Q 1/68* (1980.01)
*C07K 14/47* (1995.01) *G01N 33/50* (1980.01)

(86) International application number:
**PCT/JP2004/011585**

(87) International publication number:
**WO 2005/014813 (17.02.2005 Gazette 2005/07)**

(84) Designated Contracting States:
**AT BE BG CH CY CZ DE DK EE ES FI FR GB GR HU IE IT LI LU MC NL PL PT RO SE SI SK TR**

(30) Priority: **08.08.2003 JP 2003206948**
**06.01.2004 JP 2004000732**

(71) Applicant: **Astellas Pharma Inc.**
**Tokyo 103-8411 (JP)**

(72) Inventors:
- **Endoh, Hideki**
**Tsukuba-shi,**
**Ibaraki 3058585 (JP)**
- **Ueda, Yoshitaka**
**Tsukuba-shi,**
**Ibaraki 3058585 (JP)**

(74) Representative: **Bates, Philip Ian**
**Reddie & Grose**
**16 Theobalds Road**
**London WC1X 8PL (GB)**

# (54) NOVEL PROTEIN USABLE IN SCREENING DRUG FOR IMPROVING TYPE 2 DIABETES

(57) The present invention provides a method of screening a drug for improving type 2 diabetes. A protein CbAP40 binding to c-Cbl is found out. It is further found out that mouse CbAP40 gene shows a remarkable increase in expression amount in the muscle of diabetes model mice compared with a normal individual and glucose incorporation is inhibited by overexpressing human CbAP40 gene in a muscle-origin cell, thereby clarifying that the above protein is a factor causative of diabetic conditions. Moreover, the promoter region of human CbAP40 gene is identified and it is clarified that a transcription-inducing activity originating in this promoter region is inhibited by a thiazolidine derivative that improves insulin resistance. Based on these findings, systems for screening a substance having an effect of improving insulin resistance, in which a change of promoter activity and a change in the interaction between c-Cbl and CbAP40 are indicators, are constructed.

EP 1 652 922 A1

## Description

### Technical Field

The present invention relates to a screening method of an agent for improving type 2 diabetes. The present invention also relates to a novel polypeptide binding to c-Cbl and a polynucleotide encoding the polypeptide. Furthermore, the present invention relates to a promoter controlling the expression level of the polypeptide, an expression vector containing the polynucleotide or the promoter, and a transformant cell containing the expression vector. Moreover, the present invention relates to use of the polypeptide, the promoter, the expression vector and/or the transformant cell for screening of an agent for improving type 2 diabetes.

### Background of the Invention

Insulin is secreted from β cells in the Langerhans islet in pancreas and mainly acts on muscle, liver and adipose to allow blood glucose to be incorporated into the cells for storage and consumption to thereby decrease the blood glucose level. Diabetes mellitus is caused by functional insufficiency of insulin. The patients are grouped into two types, namely type 1 patient with disordered insulin generation or secretion and type 2 patient with difficulty in the promotion of glucose metabolism with insulin. Blood glucose levels in both types of the patients are higher than the levels in healthy persons. While blood insulin is absolutely insufficient in the type 1, insulin resistance emerges in the type 2. In other words, the incorporation or consumption of blood glucose in cells is not promoted in the type 2 despite the existence of insulin. Type 2 diabetes is one of so-called adult diseases triggered by causes such as overeating, insufficient exercise, and stress in addition to genetic disposition. In the developed countries, lately, patients of the type 2 diabetes are rapidly increased in number in accordance with the increase of calories uptake. The patients occupy 95% of diabetic patients in Japan. Therefore, the need of research works is increasing, not only about simple hypoglycemic agents as therapeutic agents of diabetes but also about the therapeutic treatment of type 2 diabetes so as to promote glucose metabolism through the amelioration of insulin resistance.

Currently, insulin injections are prescribed for the therapeutic treatment of patients of type 1 diabetes. As hypoglycemic agents to be prescribed for patients of type 2 diabetes, alternatively, there have been known sulfonyl urea-series hypoglycemic agents (SU agents) which act on pancreatic β cells to promote insulin secretion, biguanide-series hypoglycemic agents having an action on the increase of glucose utilization or the suppression of gluconeogenesis via anaerobic glycolysis and an action on the suppression of intestinal glucose absorption, and α-glucosidase inhibitor delaying sugar digestion and absorption, in addition to insulin injections. They ameliorate insulin resistance in an indirect manner. Thiazolidine derivatives as agents for directly improving insulin resistance have been used in recent years. The actions work for glucose incorporation into cells and the promotion of intracellular glucose utilization. It is described that the thiazolidine derivatives function as agonists of peroxisome proliferator activated receptor gamma (PPARγ) (see Non-Patent Reference 1). However, it is known that thiazolidine derivatives not only ameliorate insulin resistance but also have a side effect to induce edema (see Non-Patent References 2 and 3). Because the induction of edema is a serious adverse action causing cardiac hypertrophy, a more useful target molecule for pharmaceutical creation in place of PPARγ is essentially required so as to improve insulin resistance.

The signal of insulin action is transferred through an insulin receptor on cell membrane to the inside of cell. The signaling pathway for insulin action includes two pathways, namely first and second pathways (see Non-Patent Reference 4). In the first pathway, the signal is transferred from the activated insulin receptor sequentially through IRS-1, IRS-2, PI3 kinase and PDK1 to Akt1 (PKBα) or Akt2 (PKBβ), or PKCλ or PKCξ. Consequently, glucose transporter GLUT4 existing intracellularly is translocated onto cell membrane, so that extracellular glucose incorporation is promoted (see Non-Patent Reference 5). In the second pathway, meanwhile, the signal is transferred from the insulin receptor sequentially through c-Cbl and CAP to CrkII, C3G and TC10, so that glucose incorporation with GLUT4 is promoted (see Non-Patent Reference 6). However, most of the details of these insulin signal transduction pathways have not yet been elucidated. Particularly, it is not yet clearly shown as to what kind of mechanism finally works for these signals to promote cellular glucose incorporation through the glucose transporter.

c-Cbl is a signal transduction-mediating factor existing on the second insulin signaling pathway and is a proline-rich cytoplasmic protein of 120 kDa. Tyrosine in c-Cbl is transiently phosphorylated on insulin stimulation and c-Cbl is then associated with various signal transduction molecules having SH2 and SH3. For example, CAP (Cbl associated protein) is an adaptor protein existing on the second insulin signaling pathway and is highly expressed in insulin-responsive tissues such as liver, skeletal muscle, kidney and heart (see Non-Patent Reference 7). CAP is bound through the SH3 domain at the C terminus thereof to c-Cbl. In response to insulin signaling, the CAP/c-Cbl complex promotes the translocation of the glucose transporter GLUT4 through the CrkII-C3G complex and TC10 to cell membrane. It is reported that CAP in which SH3 as the binding domain to c-Cbl is deleted never affects PI3 kinase activity but inhibits cellular glucose incorporation (see Non-Patent Reference 8). Additionally, it is also known that CAP expression is activated by thiazolidine derivatives as PPARγ agonists improving insulin resistance. Based on these facts, it is understood that c-Cbl is a signal transduction-mediating factor functioning through CAP binding for intracellular glucose incorporation and that the inhibition of the function causes insulin resistance by blocking insulin signaling in the downstream of CAP (see Non-Patent Reference 9). Thus, it is believed that insulin signal transduction through c-Cbl is inhibited by some mechanism in the cells of patients of type 2 diabetes having insulin resistance (see Non-Patent Reference 9). However, no molecule downregulating the activity responsible for insulin signal transduction by direct interaction with c-Cbl has been known so far.

(Non-patent reference 1) *The Journal of Biological Chemistry,* (USA), 1995, Vol. 270, p. 12953-12956
(Non-patent reference 2) *Diabetes Frontier,* (USA), 1999, Vol. 10, p. 811-818
(Non-patent reference 3) *Diabetes Frontier,* (USA), 1999, Vol. 10, p.819-824
(Non-patent reference 4) *The Journal of Clinical Investigation,* (USA), 2000, Vol. 106, No. 2, p. 165-169
(Non-patent reference 5) *The Journal of Biological Chemistry,* (USA), 1999, Vol. 274, No. 4, p. 1865-1868
(Non-patent reference 6) *Nature*, (UK), 2001, Vol. 410, No. 6831, p. 944-948
(Non-patent reference 7) *Molecular and Cellular Biology,* (USA), 1998, Vol. 18, No. 2, p. 872-879
(Non-patent reference 8) *The Journal of Biological Chemistry*, (USA), 2001, Vol. 276, No. 9, p. 6065-6068
(Non-patent reference 9) *The Journal of Biological Chemistry,* (USA), 2000, Vol. 275, No. 13, p. 9131-9135

## Disclosure of the Invention

An object of the present invention is to provide a screening method of an agent for improving type 2 diabetes.

The inventors identified a protein binding to c-Cbl by the yeast two-hybrid system. As a result, a protein binding to c-Cbl, namely human CbAP40 (Cbl associated protein 40) was found. Additionally, the inventors found that the expression of the gene encoding the protein was localized in skeletal muscle as one of insulin responsive tissues. Furthermore, the inventors obtained mouse CbAP40 gene and protein and clarified that the protein binds to c-Cbl. Still further, the inventors found that the mouse CbAP40 gene was significantly expressed in the muscle of diabetic model mice, in comparison with normal mice and that the human CbAP40 gene inhibited glucose incorporation when expressed highly in a muscle-derived cell. Thus, the inventors found that the protein was a causative factor of diabetic conditions and provided a novel screening tool of an agent for improving type 2 diabetes. The inventors additionally identified the promoter region of the human CbAP40 gene and then found that the transcription induction activity derived from the promoter was suppressed by thiazolidine derivatives which is known to improve insulin resistance. Based on these findings, the inventors demonstrated that an effect on the amelioration of insulin resistance was obtained by suppressing the CbAP40 promoter-derived transcription induction activity. Based on these findings, the inventors constructed a screening system of a substance having effects on the therapeutic treatment of type 2 diabetes, using the promoter activity as an indicator.

That is, the present invention relates to a screening method, a polypeptide, a polynucleotide, an expression vector containing the polynucleotide, a cell transformed with the expression vector and use thereof, described below.

[1] A method for assaying whether or not a test substance is capable of inhibiting promoter activity of a polynucleotide of any one of the following (i) to (iv), which comprises:

(1) a step of bringing a test substance into contact with a cell transformed with an expression vector containing a polynucleotide which consists of (i) the nucleotide sequence represented by SEQ ID NO:3, (ii) the nucleotide sequence represented by positions 1364 to 3119 in the nucleotide sequence represented by SEQ ID NO:3, or (iii) the nucleotide sequence represented by positions 2125 to 3119 in the nucleotide sequence represented by SEQ ID NO:3; or a polynucleotide which comprises (iv) a nucleotide sequence in which 1 to 10 nucleotides are deleted, substituted and/or inserted in any one of the nucleotide sequences represented by (i) to (iii), and which has promoter activity of a polypeptide consisting of the amino acid sequence represented by SEQ ID NO:2 or SEQ ID NO:26; and
(2) a step of detecting the promoter activity.

[2] A method for screening a substance capable of suppressing expression of the polypeptide according to [1], which comprises:

an analysis step by the method according to [1]; and
a step of selecting a substance capable of inhibiting the promoter activity.

[3] A method for screening an agent for improving type 2 diabetes by the method according to [2].

[4] A polynucleotide consisting of (1) the nucleotide sequence represented by SEQ ID NO:3, (2) the nucleotide sequence represented by positions 1364 to 3119 in the nucleotide sequence represented by SEQ ID NO:3, or (3) the nucleotide sequence represented by positions 2125 to 3119 in the nucleotide sequence represented by SEQ ID NO:3; or a polynucleotide which consists of (4) a nucleotide sequence in which 1 to 10 nucleotides are deleted, substituted, inserted and/or added in any one of the nucleotide sequences represented by (1) to (3), and which has promoter activity of the polypeptide according to [1].

[5] A method for assaying whether or not a test substance is capable of inhibiting binding of a polypeptide to c-Cbl, which comprises:

a step of bringing the polypeptide and c-Cbl into contact with a test substance, wherein the polypeptide comprises (1) the amino acid sequence represented by SEQ ID NO:2 or SEQ ID NO:26, (2) an amino acid sequence in which 1 to 10 amino acids are deleted, substituted and/or inserted in the amino acid sequence represented by SEQ ID NO:2 or SEQ ID NO:26, or (3) an amino acid sequence having 90% or more homology to the amino acid sequence represented by SEQ ID NO:2 or SEQ ID NO:26, and is capable of inhibiting glucose incorporation by binding to c-Cbl and/or overexpression; and
a step of detecting binding of the polypeptide to c-Cbl.

[6] A method for screening a substance capable of inhibiting binding of the polypeptide according to [5] to c-Cbl, which comprises:

an assaying step by the method according to [5], and
a step of selecting a substance capable of inhibiting the binding.

[7] A method for screening an agent for improving type 2 diabetes by the method described in [6].

[8] A polypeptide which comprises the amino acid sequence represented by SEQ ID NO:2 or SEQ ID NO:26, or an amino acid sequence in which 1 to 10 amino acids are deleted, substituted and/or inserted in the amino acid sequence represented by SEQ ID NO:2 or SEQ ID NO:26, and which is capable of inhibiting glucose incorporation by binding to c-Cbl and/or overexpression.

[9] A polypeptide consisting of the amino acid sequence represented by SEQ ID NO:2 or SEQ ID NO:26.

[10] A polynucleotide encoding a polypeptide which consists of the amino acid sequence represented by SEQ ID NO:26, or an amino acid sequence in which 1 to 10 amino acids are deleted, substituted, inserted and/or added in the amino acid sequence represented by SEQ ID NO:26, and which is capable of inhibiting glucose incorporation by binding to c-Cbl and/or overexpression.

[11] An expression vector containing the polynucleotide according to [4] or [10].

[12] A cell transformed with the expression vector according to [11].

[13] A screening tool of an agent for improving type 2 diabetes, which comprises

(1) the polypeptide according to [8], (2) a polynucleotide encoding the polypeptide according to [8] or the polynucleotide of any one of (i) to (iv) according to [1], or (3) a polynucleotide encoding the polypeptide according to [8] or a cell transformed with an expression vector containing the polynucleotide of any one of (i) to (iv) according to [1].

[14] Use of (1) the polypeptide according to [8], (2) a polynucleotide encoding the polypeptide according to [8] or the polynucleotide of any one of (i) to (iv) according to [1], or (3) a polynucleotide encoding the polypeptide according to [8] or a cell transformed with an expression vector containing the polynucleotide of any one of (i) to (iv) according to [1] for screening of an agent for improving type 2 diabetes.

Preferably, the method for screening an agent for improving type 2 diabetes as described in [3] or [7] further includes an assaying step of improving function of type 2 diabetes.

The agent for improving type 2 diabetes as obtained according to the screening method of the present invention is particularly preferable as an agent for improving insulin resistance and/or an agent for improving glucose metabolism. Additionally, the screening tool of an agent for improving type 2 diabetes of the present invention is particularly preferable as a screening tool of an agent for improving insulin resistance and/or an agent for improving glucose metabolism.

The sequence which is the same as the polypeptide consisting of the sequence represented by SEQ ID NO: 26 of the present invention has not yet been known. Prior to the priority date of the present application (August 8, 2003), the same sequence as the amino acid sequence represented by SEQ ID NO:2 as one sequence of the polypeptides of the present invention was listed as Accession No. AK091037 in the sequence database GenPept. Prior to the priority date of the present application (January 6, 2004), an amino acid sequence in which four amino acids are substituted and 103 amino acids are added in the amino acid sequence represented by SEQ ID NO:26 as one sequences of the polypeptide of the present invention was listed as Accession No. AK044445 in the sequence database GenPept. However, there is no information telling that these peptides were actually obtained or no detailed specific information showing how these peptides can be obtained. Additionally, specific use of the polypeptides is not described. It is described on the database that the polypeptide sequence of Accession No. AK044445 is putative. The present inventors first prepared the polypeptide of the present invention and then first found that the activation of the expression of the polypeptide of the present invention and the interaction thereof with c-Cbl caused diabetic conditions. Additionally, the inventors first provided the screening method of the present invention using binding of the polypeptide of the present invention to c-Cbl.

Prior to the priority date of the application, the sequence database GenBank lists a sequence of 159246 nucleotides partially including a sequence in which one nucleotide is substituted in the nucleotide sequence of 3119 nucleotides represented by SEQ ID NO:3 under Accession No. AL590235. The database merely discloses the sequence. Thus, the specific use thereof is not described anywhere. Any polynucleotide identical to the polynucleotide of the nucleotide sequence represented by SEQ ID NO:3, the nucleotide sequence of positions 1364 to 3119 in the nucleotide sequence represented by SEQ ID NO:3, or the nucleotide sequence of positions 2125 to 3119 in the nucleotide sequence represented by SEQ ID NO:3 is not known. The inventors first provided the screening method of the present invention using the promoter activity of the polynucleotide of the present invention as an indicator.

## BRIEF DESCRIPTION OF THE DRAWINGS

Fig. 1 is a chart depicting the human CbAP40 expression in a culture cell. Lane 1 shows the case of a vacant vector introduction and Lane 2 shows the case of pcDNA-CbAP40 introduction. Lane 3 shows a molecular marker.

Fig. 2 comparatively depicts the CbAP40 gene expression in muscle tissues of normal mice C57BL/6J and m+/m+ and type 2 diabetic model mice KKA$^y$/Ta and db/db in bar graphs. The vertical axis in the drawing shows the relative expression level in mouse muscle. The expression level in C57BL/6J is expressed as 1.

Fig. 3 shows the glucose incorporation in the muscle cell involving CbAP40 overexpression. The ordinate shows the incorporation (cpm) of 2-deoxy-D-glucose. The abscissa shows the insulin concentration in a culture medium at the time of assay. The solid bars show the results in muscle cells in which CbAP40 was highly expressed, while blank bars show the results in muscle cells with which control virus was infected.

Fig. 4 shows the transcription induction activity of CbAP40 promoter and the pioglitazone action on the suppression thereof. The numerical figures in the ordinate in the drawing express luciferase activity. The numerical figures in the vertical axis in the drawing express a pioglitazone concentration (μM).

## Best Mode for Carrying out the Invention

The present invention is now described in detail hereinbelow.

<Polypeptide of the Present Invention>

The polypeptide of the present invention includes:

(1) a polypeptide consisting of the amino acid sequence represented by SEQ ID NO:2;

(2) a polypeptide comprising:

the amino acid sequence represented by SEQ ID NO:2, or
an amino acid sequence in which 1 to 10 (preferably 1 to 7, more preferably 1 to 5, and still preferably 1 to 3) amino acids are deleted, substituted and/or inserted in the amino acid sequence represented by SEQ ID NO: 2, and
being capable of inhibiting glucose incorporation by binding to c-Cbl and/or overexpression (preferably inhibiting glucose incorporation by binding to c-Cbl and overexpression) (hereinafter referred to as human functionally equivalent mutant);

(3) a polypeptide consisting of the amino acid sequence represented by SEQ ID NO:26; and

(4) a polypeptide comprising:

the amino acid sequence represented by SEQ ID NO:26, or
an amino acid sequence in which 1 to 10 (preferably 1 to 7, more preferably 1 to 5, and still more preferably 1 to 3) amino acids are deleted, substituted and/or inserted in the amino acid sequence represented by SEQ ID NO:26, and

being capable of inhibiting glucose incorporation by binding to c-Cbl and/or overexpression (preferably inhibiting glucose incorporation by binding to c-Cbl and overexpression) (hereinafter referred to as mouse functionally equivalent mutant).

Additionally, the origin of the human or mouse functionally equivalent mutant of the present invention is not limited to humans or mice. The mutant includes not only human or mouse mutants of the amino acid sequence represented by SEQ ID NO:2 or SEQ ID NO:26 but also those derived from vertebrates (for example, rat, rabbit, horse, sheep, dog, monkey, cat, bear, pig, chicken, *etc.)* other than humans and mice. Furthermore, any polypeptide grouped in any one of (1) to (4) is satisfactory as the polypeptide, and is not limited to naturally occurring polypeptides. The polypeptide includes polypeptides prepared by artificial modification based on the amino acid sequence represented by SEQ ID NO: 2 or SEQ ID NO:26 in a genetic engineering manner. Naturally occurring polypeptides, particularly polypeptides from vertebrates, are more preferable.

The phrase "binding to c-Cbl" means that the polypeptide (preferably, the polypeptide encoded by the nucleotide sequence under Accession No. X57111 in the GenBank) binds to c-Cbl. Whether or not the polypeptide is capable of "binding" to c-Cbl can be determined by the following method.

A part or full length of a subject polypeptide for the examination about the possibility of binding or a part or full length thereof after fusing with a tag such as GST, Flag, or His is expressed in a cell. The cell is preferably an insulin-responsive cell. Specifically, the cell is a cell derived from adipocytes, hepatocytes or skeletal muscle cells. The c-Cbl protein and a protein binding to the protein can be concentrated from the cell by immunoprecipitation using an anti-c-Cbl antibody. The concentrated solution of the resulting c-Cbl and the binding protein is subjected to polyacrylamide gel electrophoresis according to a known method to separate c-Cbl and the binding protein. Whether or not the subject polypeptide can bind to c-Cbl can be confirmed by Western blotting using such an antibody. The antibody for use herein is an antibody against the subject polypeptide, or an antibody against the subject polypeptide as prepared on the basis of a partial sequence thereof, or an antibody recognizing the tag described above.

Additionally, a combination of the *in vitro* pull-down method [*Experimental engineering (Jikken Kogaku*), Vol. 113, No. 6, 1994, p. 528, Matsushime, *et al.*] using an extract of a cell involving the expression of the subject polypeptide or a protein mixture solution prepared by *in vitro* transcription and translation, and c-Cbl protein purified after the addition of a tag, such as GST, together with the Western blotting described above, can also be used for the detection of the binding of the subject polypeptide to c-Cbl. Preferably, a protein mixture solution prepared by direct *in vitro* transcription and translation of the subject protein from the plasmid for expressing the subject protein as described in Example 9 by using in vitro translation kit (for example, TNT kit, Promega) is used to detect the binding. More preferably, the binding of the subject polypeptide to c-Cbl can be detected by the method described in Example 9.

The phrase "inhibiting glucose incorporation by overexpression" means that overexpression of a certain polypeptide inhibits glucose incorporation in comparison with the no-overexpression of the polypeptide. Whether or not "glucose incorporation is inhibited" can be confirmed by the following method. A cell (for example, muscle cell L6) is transformed with an expression vector containing the polynucleotide encoding the subject polypeptide. Whether or not the subject polypeptide is highly expressed (overexpressed) in the cell by the transformation can be confirmed by Western blotting using the cell extract solution and an antibody capable of detecting the subject polypeptide or by real-time PCR using a primer specifically detecting a polynucleotide encoding the subject polypeptide, or the like. Whether or not the subject polypeptide inhibits glucose incorporation is confirmed by measuring glucose incorporated into cells, using a cell which overexpresses or does not overexpress the polypeptide. When the glucose incorporation in the cell which overexpresses the subject polypeptide decreases in comparison with that in the cell which does not overexpress the polypeptide, it can be determined that the subject polypeptide inhibits glucose incorporation by the overexpression.

Preferably, the method described in Example 6 can confirm whether or not the subject polypeptide inhibits glucose incorporation by the overexpression.

The polypeptide of the present invention is described hereinabove. The polypeptide consisting of the amino acid sequence represented by SEQ ID NO:2 or SEQ ID NO:26 and the human or mouse functionally equivalent mutants of the present invention are collectively referred to as "the polypeptide of the present invention" hereinbelow. In "the polypeptide of the present invention", a protein which is the polypeptide consisting of the amino acid sequence of ID NO:2 is referred to as "human CbAP40 protein" and a protein which is the polypeptide consisting of the amino acid sequence represented by SEQ ID NO:26 is referred to as "mouse CbAP40 protein".

The polypeptide of the present invention is most preferably the polypeptide consisting of the amino acid sequence represented by SEQ ID NO:2 or SEQ ID NO:26 or, a polypeptide comprising the amino acid sequence represented by SEQ ID NO:2 or SEQ ID NO:26 among the human or mouse functionally equivalent mutants,.

The inventors found that CbAP40 as one type of the polypeptide of the present invention could bind to c-Cbl (Examples 1 and 9) and additionally that glucose incorporation decreased when the gene encoding the human CbAP40 was highly expressed in a muscle cell (Example 6). Therefore, the inventors considered that CbAP40 suppressed the c-Cbl function in the insulin signal transduction, and then found that a substance capable of inhibiting the binding of the polypeptide of the present invention to c-Cbl would be a substance for improving glucose incorporation, namely an agent for improving type 2 diabetes. The polypeptide of the present invention is useful as a screening tool for the method for screening the substance capable of inhibiting the binding (namely, an agent for improving type 2 diabetes, particularly a substance for improving glucose incorporation).

<Process for preparing the polynucleotide of the present invention and polynucleotides described in this specification>

The polynucleotide of the present invention includes:

[1] a polynucleotide consisting of a nucleotide sequence encoding the mouse CbAP40 protein or a polypeptide which is a mouse functionally equivalent mutant (hereinafter referred to as mouse type polynucleotide); and
[2] a polynucleotide consisting of (1) the nucleotide sequence represented by SEQ ID NO:3, (2) a nucleotide sequence of positions 1364 to 3119 in the nucleotide sequence represented by SEQ ID NO:3; or (3) a nucleotide sequence of positions 2125 to 3119 in the nucleotide sequence represented by SEQ ID NO:3, or a polynucleotide comprising (4) a nucleotide sequence in which 1 to 10 nucleotides are deleted, substituted and/or inserted in any one of the nucleotide sequences represented by (1) to (3), and having promoter activity of the mouse CbAP40 protein or a polypeptide which is a mouse functionally equivalent mutant (hereinafter referred to as promoter type polynucleotide).

The mouse type polynucleotide may satisfactorily have a nucleotide sequence derived from any species, so long as the nucleotide sequence encodes the mouse CbAP40 or a polypeptide which is a mouse functionally equivalent mutant. The mouse type polynucleotide is preferably a polynucleotide consisting of the nucleotide sequence encoding the mouse CbAP40 and is most preferably the polynucleotide represented by SEQ ID NO:25. In the promoter type polynucleotide, most preferable is a polynucleotide consisting of the nucleotide sequence represented by positions 2125 to 3119 in the nucleotide sequence represented by SEQ ID NO:3.
The mouse type polynucleotide includes any mutant, so long as the mutant encodes the mouse CbAP40 protein and a polypeptide which is a mouse functionally equivalent mutant. The promoter type polynucleotide includes a polynucleotide consisting of (1) the nucleotide sequence represented by SEQ ID NO:3, (2) the nucleotide sequence represented by positions 1364 to 3119 in the nucleotide sequence represented by SEQ ID NO:3, or (3) the nucleotide sequence represented by positions 2125 to 3119 in the nucleotide sequence represented by SEQ ID NO:3, or any mutant consisting of (4) a nucleotide sequence in which 1 to 10 nucleotides are deleted, substituted and/or inserted in any one of the nucleotide sequences represented by (1) to (3), and having promoter activity of the human or mouse CbAP40 protein or the polypeptide which is a human or mouse functionally equivalent mutant. More specifically, naturally occurring mutants, mutants which do not exist naturally, and mutants having deletion, substitution, addition and insertion are included. The mutation described above may be sometimes caused by spontaneous mutagenesis from natural origins but may also be induced by artificial modification. The cause and measure for the mutation of the polynucleotide may be any cause and measure in accordance with the present invention. The artificial measure for preparing the mutant includes, for example, genetic engineering techniques such as nucleotide-directed substitution method *(Methods in Enzymology*, (1987) 154, 350, 367-382), and chemical synthesis measure such as the phosphate triester method and the phosphoramidite method *(Science,* 150, 178, 1968). It is possible to obtain DNA involving a desired nucleotide substitution by a combination thereof. Otherwise, it is also possible to generate the substitution of a non-specified nucleotide in a DNA molecule by the repetitive manipulation of the PCR method or by the presence of manganese ion and the like in the reaction solution.
The promoter type polynucleotide of the present invention and the polynucleotide encoding the polypeptide of the present invention can be prepared and obtained easily by general genetic engineering techniques on the basis of the sequence information disclosed in the present invention.
The promoter of the present invention and the polynucleotide encoding the polypeptide of the present invention can be obtained, for example, as follows. With no limitation to the methods described below, however, these polynucleotides can be obtained by known procedures (*Molecular Cloning,* Sambrook, J., *et al*., Cold Spring Harbor Laboratory Press, 1989, *etc.).*
For example, the methods include (1) a method using PCR, (2) a method using ordinary genetic engineering technique (namely, a method of selecting a transformant containing a desired amino acid sequence from transformants transformed with cDNA library), (3) a chemical synthesis method, and the like. The respective methods can be carried out in the same manner as described in WO01/34785.
In the method using PCR, the polynucleotide described in this specification can be prepared, for example, by procedures described in the above patent reference, the section "Mode for Carrying Out the Invention", 1) Production method of protein gene, a) First production method. In the description, the phrase "human cell or tissue having the ability to produce the mouse protein capable of the invention" includes, for example, human skeletal muscle. A mRNA is extracted from the human skeletal muscle. Then, the mRNA is subjected to reverse transcription in the presence of random primers or oligo dT primers to synthesize a first strand cDNA. Using the obtained first cDNA, a polymerase chain reaction (PCR) is carried out by using two types of primers including a partial region of the objective gene to obtain the polynucleotide of the present invention or a part thereof. More specifically, the polynucleotide encoding the polypeptide of the present invention and/or the promoter type polynucleotide of the present invention can be prepared, for example, by the method described in Example 1, 7 or 8.

In the method using ordinary genetic engineering technique, for example, the polynucleotide encoding the polypeptide of the present invention and/or the promoter type polynucleotide of the present invention can be prepared, for example, by procedures described in the patent reference, the section "Mode for Carrying Out the Invention", 1) Production method of protein gene, b) Second production method.

In the chemical synthesis method, the polynucleotide encoding the polypeptide of the present invention and/or the promoter type polynucleotide of the present invention can be prepared, for example, by procedures described in the patent reference, the section "Mode for Carrying Out the Invention", 1) Production method of protein gene, c) Third production method, d) Fourth production method.

A substance capable of suppressing expression of the polypeptide of the present invention can be screened by analyzing whether or not a test compound inhibits the promoter activity of the present invention using the promoter type polynucleotide of the present invention. The inventors found that human CbAP40 as one type of the polypeptide of the present invention inhibited glucose incorporation (Example 6) and that thiazolidine derivatives which are known to ameliorate insulin resistance suppressed the transcription induction activity derived from the promoter of the present invention (Example 7). These facts indicate that a substance capable of suppressing expression of the polypeptide of the present invention improves the inhibition of glucose incorporation and is useful as an agent for improving type 2 diabetes, particularly an agent for improving insulin resistance and/or an agent for improving glucose metabolism. Therefore, the promoter of the present invention can be used as a screening tool of the agent for improving type 2 diabetes, particularly an agent for improving insulin resistance and/or an agent for improving glucose metabolism.

The polypeptide of the present invention, for example, mouse CbAP40, can be prepared from the mouse type polynucleotide of the present invention.

<Production method of the polypeptide of the present invention>

The present invention includes a method for producing the polypeptide of the present invention, which comprises culturing a cell transformed with an expression vector into which the polynucleotide encoding the polypeptide of the present invention is introduced.

The polynucleotide encoding the polypeptide of the present invention as obtained in the manner described above can be connected to the downstream of an appropriate promoter by the method described in "*Molecular Cloning,* Sambrook, J., *et al*., Cold Spring Harbor Laboratory Press, 1989" and the like to thereby allow the expression of the polypeptide of the present invention in *in vitro* or in a test cell.

Specifically, the polypeptide of the present invention can be expressed by gene transcription and translation in a cell-free system by adding a polynucleotide containing a specific promoter sequence to the upstream of the initiation codon of the polypeptide of the present invention and using the resulting polynucleotide as a template.

Otherwise, the polypeptide of the present invention can be expressed in cells by inserting the polynucleotide encoding the polypeptide of the present invention into an appropriate plasmid vector and introducing the polynucleotide in the form of plasmid into a host cell. Still otherwise, a cell in which such a construct is integrated into the chromosome DNA may be obtained and used therefor. More specifically, a fragment containing the isolated polynucleotide is again integrated into an appropriate plasmid vector to thereby transform eukaryotic and prokaryotic host cells. Furthermore, the polypeptide of the present invention can be expressed in the individual host cells by introducing an appropriate promoter and a sequence responsible for gene expression into these vectors. The host cells are not particularly limited, and include host cells in which the expression of the polypeptide of the present invention can be assayed at mRNA level or at protein level. More preferably, a muscle-derived cell in which endogenous CbAP40 is abundant is used as the host cell.

The method for transforming the host cell and expressing the gene is carried out for example according to the method described in the above patent reference, the section "Mode for Carrying Out the Invention", 2) Methods for the production of the vector of the invention, the host cell of the invention and the recombinant protein of the invention. The expression vector is not particularly limited, so long as the expression vector contains a desired polynucleotide. Examples thereof include an expression vector obtained by inserting a desired polynucleotide into a known expression vector appropriately selected according to a host cell to be used. For example, the cell of the present invention can be obtained by transfecting a desired host cell with the expression vector. Specifically, for example, an expression vector for a desired protein can be obtained by integrating a desired polynucleotide in an expression vector for mammalian cell, pcDNA3.1 (Invitrogen), as described in Examples 2 or 8, and then, the expression vector is incorporated into the 293 cell using the calcium phosphate method to prepare the transformant cell of the present invention.

The desired transformant cell thus obtained can be cultured by ordinal methods. A desired protein can be produced by the culturing. As the culture medium for use in the culturing, various culture media for routine use are appropriately selected in a manner dependent on the host cell selected. For the 293 cell, for example, the Dulbecco's modified Eagle minimum essential culture medium (DMEM) to which serum components such as fetal bovine serum (FBS) and G418 are added can be used.

The polypeptide of the present invention produced in the cell can be detected, assayed and purified by culturing the cell of the present invention. The polypeptide of the present invention can be detected and purified by Western blotting using an antibody capable of binding to the polypeptide of the present invention or by immunoprecipitation. Otherwise, the polypeptide of the present invention can be expressed as a protein fused to an appropriate tag protein such as glutathione-S-transferase (GST), protein A, β-galactosidase, and maltose-binding protein (MBP) to detect the polypeptide of the present invention by Western blotting using an antibody specific to these tag proteins or by immunoprecipitation and to purify the polypeptide using the tag proteins. More specifically, purification can be carried out by using the tag proteins as described below.

The polypeptide of the present invention (for example, the polypeptide represented by SEQ ID NO:2 or SEQ ID NO:26) can be obtained by inserting a polynucleotide encoding the polypeptide into a vector with which a His tag is fused, specifically, for example, pcDNA3.1/V5-His-TOPO (Invitrogen) described in Examples 1 or 8 to express the polypeptide in a culture cell, and subsequently purifying the polypeptide using the His tag and then eliminating the tag moiety. For example, the human or mouse CbAP40 expression plasmid prepared by using pcDNA3.1/V5-His-TOPO in Examples 1 or 8 is designed so that the V5 and His tags can be added to the C terminus of any of the CbAP40 plasmids. Thus, using these His tags, CbAP40 protein can be purified from a culture cell expressing CbAP40 as described in Examples 2 or 8. Specifically, CbAP40 protein fused with a His tag is bound to $Ni^{2+}$-NTA-Agarose (Funakoshi) and isolated from the disrupted cell extract solution by centrifugation according to the known method (*Supplementary Issue of Experimental Medicine,* "Experimental methods of intermolecular protein interaction", 1996, No. 32, Nakahara, *et al.*). More specifically, a cell expressing the polypeptide of the present invention cultured in a culture flask (for example, a petri dish of a 10-cm diameter) is scraped from the dish by adding an appropriate volume (for example, 1 ml) of a buffer. Subsequently, the cell is centrifuged at 15,000 rpm for 5 minutes to separate the supernatant to which an appropriate amount (for example, 50 μM) of $Ni^{2+}$-NTA-Agarose diluted with an appropriate buffer is added for sufficient mixing (for example, under agitation with a rotator for 10 minutes or more). Continuously, the supernatant is separated and discarded by centrifugation (for example at 2,000 rpm for 2 minutes). An appropriate volume (for example, 0.5 ml) of a buffer adjusted to pH 6.8 is added to the precipitate, followed by centrifugation again for washing. The procedure was repeatedly carried out three times. An appropriate volume (for example, 50 μl) of 100 mM EDTA is then added to the resulting precipitate, which is then allowed to stand for 10 minutes. The polypeptide of the present invention is separated and purified by recovering the supernatant. As the buffer, for example, buffer B (8 M urea, 0.1 M $Na_2HPO_4$, 0.1 M $NaH_2PO_4$, 0.01 M tris-HCl, pH 8.0) can be used. The His tag in the purified protein molecule can be removed from the molecule, for example, by designing the His tag to be fused with the N terminus and then using TAGZyme System (Qiagen).

Alternatively, the polypeptide can also be purified by methods with no use of such tag protein, for example, by various separation procedures using the physical and chemical properties of the protein comprising the polypeptide of the present invention. Specifically, the polypeptide can be purified by using ultrafiltration, centrifugation, gel filtration, adsorption chromatography, ion exchange chromatography, affinity chromatography, and high performance liquid chromatography.

The polypeptide of the present invention can be synthesized by general chemical synthetic processes according to the amino acid sequence information represented by SEQ ID NO:2 or SEQ ID NO:26. Specifically, peptide synthetic processes by liquid phase and solid phase methods are included. The synthesis can be carried out by sequentially conjugating amino acids one by one or by synthetically preparing a peptide fragment of several amino acid residues and then conjugating the resulting peptide fragments together. The polypeptide of the present invention as obtained by these approaches can be purified by the various methods described above.

<Expression vector and cell of the present invention>

The vector of the present invention includes an expression vector containing the mouse type polynucleotide of the present invention, and an expression vector containing the promoter type polynucleotide of the present invention.

The cell of the present invention includes cells transformed with the expression vector containing the mouse type polynucleotide of the present invention (hereinafter referred to as mouse type polynucleotide-expressing cells) and cells transformed with the expression vector containing the promoter type polynucleotide of the present invention (hereinafter referred to as promoter type polynucleotide-expressing cells). Among the cells transformed with the expression vector containing the mouse type polynucleotide and the cells transformed with the expression vector containing the promoter type polynucleotide, the cells expressing the mouse type polynucleotide or the cells expressing the promoter activity of the promoter type polynucleotide are preferable as the cell of the present invention.

The cell transformed with the mouse type polynucleotide or the cell transformed with the promoter can be prepared by introducing the mouse type polynucleotide or the promoter type polynucleotide of the present invention into a host cell appropriately selected according to the purpose. The cell transformed with the mouse type polynucleotide or the cell transformed with the promoter can be prepared preferably by introducing the mouse type polynucleotide or the promoter type polynucleotide of the present invention into a vector appropriately selected according to the purpose.

For the purpose of constituting a system for analyzing the presence or absence of the inhibition of the promoter activity, for example, the cell transformed with the promoter is preferably prepared by introducing the promoter type polynucleotide of the present invention into a vector into which a reporter gene such as luciferase is introduced, as shown in Example 7. The reporter gene to be fused to the promoter region is not particularly limited, so long as it is a reporter gene generally used. Preferably, the reporter gene is an enzyme gene readily assayable in a quantitative manner. For example, the reporter gene includes bacteria transposon-derived chloramphenicol acetyltransferase gene (CAT), fire fly-derived luciferase gene (Luc), and jellyfish-derived green fluorescent protein gene (GFP). It is preferred that the reporter gene is functionally fused with the promoter type polynucleotide of the present invention. For the purpose of constructing a screening system of a substance modulating the promoter activity of the present invention, for example, cells derived from mammals (for example, humans, mouse or rat) are preferably used. Cells derived from humans are more preferably used.

The cell transformed with the mouse type polynucleotide can be used for producing the polypeptide of the present invention.

The expression vector and the cells of the present invention can be used for the screening method of the present invention (for example, the screening method of a substance controlling the promoter activity (Example 7), and a screening method using binding of the polypeptide of the present invention to c-Cbl). Accordingly, they are useful as tools for the screening.

<Screening tool of the present invention and use for screening>

The present invention includes: (1) a screening tool for an agent for improving type 2 diabetes, comprising the polypeptide of the present invention, the polynucleotide encoding the polypeptide of the present invention, the promoter type polynucleotide of the present invention or a cell transformed with an expression vector containing the polynucleotide encoding the polypeptide of the present invention or the promoter type polynucleotide of the present invention; and

(2) use of the polypeptide of the present invention, the polynucleotide encoding the polypeptide of the present invention, the promoter type polynucleotide of the present invention, or a cell transformed with an expression vector containing the polynucleotide encoding the polypeptide of the present invention or the promoter type polynucleotide of the present invention for screening of an agent for improving type 2 diabetes.

In this specification, the term "screening tool" means a substance for use in screening (specifically, the polypeptide, the polynucleotide and the cell for use in screening). The term "screening tool for an agent for improving type 2 diabetes" means a cell, a polynucleotide or a polypeptide as subjects in contact to a test substance according to the screening method of the present invention, so as to screen for an agent for improving type 2 diabetes (particularly, an agent for improving insulin resistance and/or an agent for improving glucose metabolism). The present invention also includes use of the polypeptide, the polynucleotide or the cell of the present invention for screening of an agent for improving type 2 diabetes (particularly, an agent for improving insulin resistance and/or an agent for improving glucose metabolism).

<Analytical method or screening method of the present invention>

The inventors found that one type of the polypeptide of the present invention, namely CbAP40, could bind to c-Cbl (Examples 1 and 9), that the expression of CbAP40 increased in a diabetic model mouse (Example 5), and that glucose incorporation decreased when the gene encoding human CbAP40 protein was highly expressed in muscle cells (Example 6). Therefore, the inventors found that a substance capable of inhibiting the binding of the polypeptide of the present invention to CbAP40 would be a substance for improving glucose incorporation. Additionally, the inventors found that the transcription induction activity derived from the promoter of the polypeptide of the present invention was suppressed by thiazolidine derivatives which are known to ameliorate insulin resistance (Example 7). These facts indicated that a substance capable of improving type 2 diabetes could be screened for, using the promoter activity as an indicator.

That is, the analytical method or screening method of the present invention includes a screening method of a substance capable of improving type 2 diabetes (a substance capable of improving insulin resistance and/or substance capable of improving glucose metabolism, in particular), using the change of the interaction between the polypeptide of the present invention and the c-Cbl protein as an indicator. Additionally, the analytical method or screening method in accordance with the present invention encompasses a screening method of a substance capable of improving type 2 diabetes (a substance capable of improving insulin resistance and/or substance capable of improving glucose metabolism, in particular), using the promoter type polynucleotide of the present invention so as to use the change of the promoter activity as an indicator.

The polypeptide for use in the screening of the present invention using the interaction with c-Cbl protein includes the polypeptide of the present invention or homologous peptides thereof. Polypeptides which consist of an amino acid sequences having 90% or more homology to the amino acid sequence represented by SEQ ID NO:2 or SEQ ID NO:26 and are proteins capable of binding to c-Cbl are referred to as homologous polypeptides. The homologous polypeptide in this specification is not particularly limited, so long as it is a polypeptide which consists of an amino acid sequence having 90% or more homology to the amino acid sequence represented by SEQ ID NO:2 or SEQ ID NO:26 and which is capable of binding to c-Cbl. The homologous polypeptide is a polypeptide consisting of an amino acid sequence having preferably 95% or more homology, more preferably 98% or more homology, to the amino acid sequence represented by SEQ ID NO:2 or SEQ ID NO:26.

In this specification, the term "homology" means the value of the extent of similarity obtained by using default parameters for retrieval on the Clustal program (Higgins and Sharp, *Gene*, 73, 237-244, 1998: Thompson, *et al., Nucl. Acids Res., 22,* 4673-4680, 1994) (Clusta V). The parameters are as follows.

As pairwise alignment parameters:

K tuple 1,
Gap Penalty 3,
Window 5,
Diagonals Saved 5.

The polypeptide for use in the screening of the present invention (namely, the polypeptide and homologous peptide in accordance with the present invention) is referred to as screening polypeptide.

The analytical method or screening method of the present invention more specifically includes the following methods.

First, methods using the promoter of the present invention include:

<1> a method for assaying whether or not a test substance is capable of inhibiting promoter activity of the present invention, which comprises:

(1) a step of bringing a test substance into contact with a cell expressing the promoter of the present invention, and
(2) a step of detecting the promoter activity.

<2> a method for screening a substance capable of suppressing expression of the polypeptide of the present invention or an agent for improving type 2 diabetes, which comprises:

an analytical step by the method described in <1>, and
a step of selecting a substance capable of inhibiting the promoter activity.

Second, methods using binding of the polypeptide of the present invention to c-Cbl include:

<3> a method for assaying whether or not a test substance inhibits binding of the screening polypeptide to c-Cbl, which comprises:

a step of bringing the screening polypeptide of the present invention and c-Cbl into contact with a test substance, and
a step of detecting the binding between the polypeptide and c-Cbl.

<4> a method for screening of a substance capable of inhibiting binding of the screening polypeptide of the present invention to c-Cbl or an agent for improving type 2 diabetes, which comprises:

an analytical step according to the method described in <3>, and
a step of selecting a substance capable of inhibiting the binding.

One of the modes of the methods using the promoter of the present invention is the reporter gene assay system. The reporter gene assay (Tamura, *et al*., *Research Method of Transcription Factor* (*Tensha In-shi Kenkyu-ho*, Yodosha Press) is a method for detecting the regulation of gene expression using the expression of a reporter gene as a marker. Generally, gene expression is regulated by a region called promoter region existing in the 5' upstream region. The gene expression level at the transcription stage can be estimated by assaying the promoter activity. When a test substance activates the promoter, the transcription of the reporter gene arranged downstream the promoter region is activated. In such manner, the promoter activation, namely the action to activate the expression, can be replaced with the expression of the reporter gene, so as to detect such an action. Accordingly, the action of a test substance on the regulation of the expression of the polypeptide of the present invention can be replaced with the expression of the reporter gene, so as to detect the action by the reporter gene assay using the promoter type polynucleotide of the present invention. The "reporter gene" fused with the promoter type polynucleotide of the present invention (for example, a sequence consisting of the nucleotide sequence represented by SEQ ID NO:3) is not particularly limited, so long as it is a reporter gene for routine use. It is preferably an enzyme gene easily assayable in a quantitative manner. For example, the reporter gene includes bacterial chloramphenicol acetyltransferase gene (CAT), fire fly-derived luciferase gene (Luc), and jellyfish-derived green fluorescent protein gene (GFP). The reporter gene is functionally fused to the promoter type polynucleotide of the present invention, satisfactorily. By comparing the expression level of the reporter gene in the case of a test substance in contact to a cell transformed by the reporter gene fused with the promoter of the present invention with the expression level thereof in the case of no such contact, the change of the transcription induction activity depending on the test substance can be analyzed. By carrying out the steps, a substance capable of suppressing expression of the polypeptide of the present invention or an agent for improving insulin resistance and/or an agent for improving glucose metabolism can be screened for. Specifically, the screening can be carried out by the method described in Example 7.

In a system using binding of the polypeptide of the present invention to c-Cbl, specifically, a testing cell expressing a part or full length of the screening polypeptide of the present invention or a part or full length of the screening polypeptide of the present invention with which a tag such as GST, Flag or 6×His is fused, is used without or with treatment of a test substance.

The testing cell is preferably a cell responsive to insulin and specifically includes adipocyte, hepatocyte or skeletal muscle-derived cell. The c-Cbl protein and a protein binding to the protein can be concentrated from the cell by immunoprecipitation with anti-c-Cbl antibody. For the concentration process, preferably, the same test substance used for the treatment of the cell is contained in the reaction solution. The resulting concentrated solution of c-Cbl and the binding protein is subjected to polyacrylamide gel electrophoresis by a known method to assay the amount of the screening polypeptide by Western blotting using an antibody to thereby select a test substance capable of inhibiting the binding between the screening polypeptide and c-Cbl. As the antibody herein, there can be used antibodies (for example, anti-CbAP40 antibody) against the screening polypeptide, which are raised on the basis of the screening polypeptide or a partial sequence thereof or antibodies recognizing the tags described above.

Additionally, a test substance capable of inhibiting binding of c-Cbl to the screening polypeptide can be selected using cell extract solutions involving the expression of the screening polypeptide where a test substance is added or not added in combination with the *in vitro* pull-down method using the c-Cbl protein having a tag such as GST after purification and with Western blotting [*Experimental engineering* (*Jikken Kogaku*), Vol. 113, No. 6, 1994, p. 528, Matsushime, *et al*.]. Otherwise, the protein as the screening polypeptide can be directly prepared from an expression plasmid of the screening polypeptide, by *in vitro* transcription and translation using TNT kit (Promega) with no use of the extract solution of the cell expressing the screening polypeptide. Using then the resulting protein mixture solution with addition or no addition of a test substance, a test substance capable of inhibiting the binding between c-Cbl and the screening polypeptide can be selected in the same way. By any of these methods, a great number of test substances can be screened by known spot Western blotting without polyacrylamide electrophoresis. According to known ELISA including adding a test substance to a lysate of a cell simultaneously expressing the screening polypeptide fused with a tag as described above and c-Cbl, screening for and selecting a test substance capable of inhibiting the binding between c-Cbl and the screening polypeptide can be carried out. Using the known two-hybrid system in mammalian cells (Clontech), c-Cbl fused to the DNA binding region of GAL4 as a bait and the screening polypeptide fused with the VP16 transactivation region as a prey were arranged to detect the existing CAT or luciferase activity to screen for and select a test substance capable of inhibiting binding of c-Cbl to the screening polypeptide from a great number of populations of test substances.

The test substance for use according to the screening method of the present invention is not particularly limited, and includes commercially available compounds (including peptides), various known compounds registered on chemical files (including peptides), compound groups obtained by the combinatorial chemistry technique (N. Terrett, *el al., Drug Discov. Today*, 4(1): 41, 1999), microbial culture supernatants, naturally occurring components derived from plants and marine organisms, animal tissue extracts, or compounds prepared by chemical or biological modifications of the compounds selected according to the screening method of the present invention (including peptides).

The action for improving type 2 diabetes can be analyzed by methods known to a person skilled in the art or by modified methods thereof. For example, a compound selected according to the screening method of the present invention is continuously administered to a diabetic model animal; then, the action of decreasing blood glucose is confirmed at an appropriate time by routine methods or the action for suppressing the blood glucose increase after oral glucose tolerance test is confirmed by routine methods to determine the presence or absence of the effect on the amelioration of type 2 diabetes. Additionally, human insulin resistance is assayed; then, the action for improving type 2 diabetes can be analyzed, using the improvement of the value as an indicator. Insulin resistance is mainly assayed in humans by two methods. One method includes assaying blood glucose level and insulin concentration after fasting, while the other method is called glucose tolerance test, including orally administering glucose solution and determining the clearance rate of glucose from blood circulation. Furthermore, the euglycemic/hyperinsulinaemia clamp method is listed as a more accurate test. At the test, the principle that blood insulin and glucose are retained at constant concentrations is used. The total amount of glucose given and the insulin concentration for use in the metabolism are assayed over time.

<Method for testing diabetes>

Using a probe hybridizing to the polynucleotide encoding the polypeptide of the present invention under stringent conditions, the expression level of the polynucleotide encoding the polypeptide of the present invention can be assayed. Using the increase of the expression level (preferably, the expression level in skeletal muscle) as an indicator, diabetes can be diagnosed. According to the method for testing diabetes, the term "stringent conditions" means conditions with no occurrence of non-specific binding, and specifically means conditions of 0.1 $\times$ SSC (saline-sodium citrate buffer) solution containing 0.1% sodium lauryl sulfate (SDS) used at a temperature of 65°C. As the probe, DNA having at least a part or the entirety of the sequence of the polynucleotide of the present invention (or a complementary sequence thereto) and a chain length of at least 15 bp is used.

In the method for detecting diabetes, the probe and a test substance are put in contact together to analyze the probe bound to the polynucleotide (for example, mRNA or cDNA derived from mRNA) encoding the polypeptide of the present invention by known analytical methods (for example, northern blotting) to detect the occurrence of diabetes. The expression level can be analyzed additionally by applying the probe to DNA chip. When the amount of the bound probe, namely the amount of the polynucleotide encoding the polypeptide of the present invention, increases in comparison with the amount in normal subjects, the diagnosis of diabetes can be established.

The method for assaying the expression level of the polynucleotide encoding the polypeptide of the present invention includes a method for assaying the expression level by the detection of the polypeptide of the present invention. Examples of such a test method include Western blotting, immunoprecipitation and ELISA, using an antibody allowing a test sample to bind to the polypeptide of the present invention, preferably an antibody specifically binding to the polypeptide of the present invention. For assaying the amount of the polypeptide of the present invention as contained in a test sample, the polypeptide of the present invention can be used as an internal standard. The polypeptide of the present invention is useful for preparing an antibody binding to the polypeptide of the present invention. When the amount of the polypeptide of the present invention increases in comparison with that in normal subjects, the diagnosis of diabetes can be established.

The present invention is now described in detail in the following Examples. However, the present invention is not limited to the Examples. Unless otherwise stated, the present invention can be carried out by known methods (*Molecular Cloning,* Sambrook, J., *et al*., Cold Spring Harbor Laboratory Press, 1989, *etc*.). Additionally, the present invention may also be carried out using commercially available reagents and kits according to the instructions of such products.

Example 1: Cloning of gene of c-Cbl-binding molecule CbAP40 and construction of expression vector

(1) Cloning of c-Cbl gene

Using oligonucleotides represented by SEQ ID NOs:4 and 5 (for 5' side) and those represented by SEQ ID NOs:6 and 7 (for 3' side), as designed on the basis of the cDNA sequence encoding the full length mouse c-Cbl as Accession No. X57111 in the gene database GenBank as primers and mouse skeletal muscle cDNA as a template, PCR was carried out using DNA polymerase (Pyrobest DNA polymerase; Takara Shuzo) under conditions of thermal denaturation at 95°C for 3 minutes, a cycle of 98°C for 10 seconds, 60°C for 30 seconds and 74°C for 1.5 minutes as repeated forty times, and treatment at 74°C for 7 minutes. DNA fragments of about 1.3 kbp and about 1.5 kbp thus prepared were individually inserted into the *Eco*RV recognition site of a plasmid pZErO™-2.1 (Invitrogen) to subclone the 5' side and 3' side of the mouse c-Cbl cDNA. Any of the gene fragments contains the single *Bam*HI recognition site existing on the mouse c-Cbl cDNA. Utilizing the *Bam*HI recognition site, the *Kpn*I recognition site added to SEQ ID NO: 4, and the *Xho*I recognition site added to SEQ ID NO:7, the *Kpn*I-*Bam*HI fragment on the 5' side and the *Bam*HI-*Xho*I fragment on the 3' side were cleaved out from the individual subclones, and were then inserted between the *Kpn*I and *Xho*I sites of pcDNA3.1 (+) to obtain the full-length mouse c-Cbl cDNA. Furthermore, it was confirmed by using a sequencing kit (Applied BioSystems) and a sequencer (ABI 3700 DNA sequencer, Applied BioSystems) that the nucleotide sequence of the c-Cbl cDNA cloned on the vector was identical to the reported sequence.

(2) Screening by yeast two-hybrid system

According to the method described in the patent reference (WO03/06247), Example 2(2), the mouse c-Cbl cDNA was inserted in an expression vector for yeast two-hybrid system, by utilizing homologous recombination. Herein, primers represented by SEQ ID NOs:8 and 9 were designed. Using the primers and the mouse c-Cbl cDNA as a template, a c-Cbl cDNA fragment in which a 40-mer sequence required for homologous recombination was added to both the ends was obtained by PCR. The sequence in an expression vector prepared by homologous recombination was confirmed by the method described in the patent reference to Endo, *et al.*, Example 2(2). Then, an interactive factor was screened for in the human skeletal muscle library according to the same method as Example 2(3), *ibid.* A yeast cell expressing the protein binding to c-Cbl was determined. From the cell, a plasmid derived from the library was extracted. The nucleotide sequence of a gene fragment contained therein was sequenced according to the method described in Example 2(2), *ibid.* Consequently, it was confirmed that one clone containing the sequence of a region corresponding to the nucleotides at positions 934 to 1101 on the 3' side of the nucleotide sequence represented by SEQ ID NO:1. The clone contained the DNA sequence encoding the protein containing full 55 amino acid residues on the carboxyl end of the polypeptide represented by SEQ ID NO:2. The clone is capable of expressing a fusion protein containing the polypeptide of the 55 amino acid residues in yeast. Therefore, it is shown that the polypeptide represented by SEQ ID NO:2 is a protein capable of binding to c-Cbl at the part of the 55 amino acid residues on the carboxyl end thereof.

(3) Cloning of full length cDNA of human CbAP40 gene

As the consequence of (2), a library-derived plasmid containing a gene fragment containing a part of the nucleotide sequence represented by SEQ ID NO:1 was obtained, indicating the presence of a factor binding to c-Cbl. Therefore, a primer of a nucleotide sequence represented by SEQ ID NO:10 corresponding to the complementary sequence of the nucleotide sequence at positions 1079 to 1089 in the nucleotide sequence represented by SEQ ID NO: 1 was synthesized (Proligo). Using the primer, the full length cDNA was amplified from the cDNA library derived from skeletal muscle by PCR according to the method described in Example 1(4) of the patent reference (WO03/062427). PCR was carried out by DNA polymerase (LA Taq, Takara Shuzo) at 94°C (for 3 minutes) and subsequent 35-times repetition of a cycle of 94°C (for 30 seconds), 58°C (for 1.5 minutes) and 72°C (for 4 minutes). Using the resulting PCR product as a template, PCR was carried out under the same conditions. The PCR product was separated by agarose gel electrophoresis. Consequently, the amplification of a DNA fragment of about 1,200 base pairs was confirmed. Then, the DNA fragment in the reaction solution was cloned into an expression vector (pcDNA3.1/V5-His-TOPO; Invitrogen) using TOPO TA Cloning system (Invitrogen). The nucleotide sequence of the inserted DNA fragment in the resulting plasmid was determined using a primer (TOPO TA Cloning kit/Invitrogen; SEQ ID NO:11) capable of binding to the T7 promoter region on the vector, a sequencing kit (Applied BioSystems) and a sequencer (ABI 3700 DNA sequencer; Applied BioSystems). Consequently, it was confirmed that the DNA fragment was a clone containing the DNA sequence represented by SEQ ID NO:1, so that a sequence of about 70 base pairs upstream the 5' end of SEQ ID NO:1 was obtained. In view of the triplets of the DNA encoding the amino acid sequence represented by SEQ ID NO:2, no initiation codon was observed upstream the ATG (initiation codon) at the start of SEQ ID NO:1 but the triplet as the stop codon existed. Thus, the open reading frame of the gene represented by SEQ ID NO:1 was determined. The gene represented by the nucleotide sequence represented by SEQ ID NO:1 was named human CbAP40 gene.

(4) Preparation of human CbAP40 expression vector

According to the nucleotide sequence information shown by SEQ ID NO:1, a primers represented by SEQ ID NO:12 was synthesized (Proligo). Using the primer and the primers represented by SEQ ID NO:10, cDNA encoding the net human CbAP40 protein was amplified by PCR, using the plasmid obtained above in (3) as a template. These two types of DNA primers contain nucleotide sequences homologous to partial 5' and 3' sequences, respectively, of the CbAP40 gene represented by SEQ ID NO:1. PCR was carried out at 98°C (for 1 minute) and then by repeating a cycle of 98°C (for 5 seconds), 55°C (for 30 seconds) and 72°C (for 5 minutes) 35 times, using DNA polymerase (Pyrobest DNA Polymerase; Takara Shuzo). The PCR product was separated by agarose gel electrophoresis. Consequently, it was confirmed that a DNA fragment of about 1.1 kbp was amplified. Then, the DNA fragment in the reaction solution was cloned into an expression vector (pcDNA3.1/V5-His-TOPO; Invitrogen) using TOPO TA Cloning system (Invitrogen). The primer of SEQ ID NO:10 used then was designed so that the stop codon sequence of human CbAP40 might be eliminated so as to allow the vector-derived V5 epitope (derived from the V protein of paramyxovirus SV5, Southern JA, *J. Gen. Virol.,* 72, 1551-1557, 1991) and His6 tag (Lindner P, *BioTechniques,* 22, 140-149, 1997) to be successively contained in the same frame of the triplets of the CbAP40 gene on the 3' side after cloning. The nucleotide sequence of the inserted DNA fragment in the resulting plasmid was determined using a primer (TOPO TA Cloning kit/Invitrogen; SEQ ID NO:11) capable of binding to the T7 promoter region on the vector, a sequencing kit (Applied BioSystems) and a sequencer (ABI 3700 DNA sequencer; Applied BioSystems). Consequently, it was confirmed that the human CbAP40 cDNA of 1101 base pairs encoding the full human CbAP40 protein as shown as SEQ ID NO:1 was inserted as the DNA resulting from the preliminary elimination of the 3' stop codon from the DNA sequence in the expression vector pcDNA3.1/V5-His-TOPO. The expression plasmid is abbreviated hereinbelow as pcDNA-CbAP40.

Example 2: Preparation of culture cell expressing human CbAP40 protein

(1) Preparation of human CbAP40 expressing cell

The expression plasmid pcDNA-CbAP40 prepared above in Example 1 (4) or vacant vector (pcDNA3.1) (Invitrogen) was introduced into the 293 cell. The 293 cell was cultured in a 2 ml of the minimum essential culture medium DMEM (GIBCO) containing 10% fetal calf serum in each well in a 6-well culture plate (well diameter of 35 mm) until the cell reached 70% confluence. pcDNA-CbAP40 (3.0 μg/well) was transiently introduced into the cell by the calcium phosphate method (Graham, *et al.*, *Virology,* 52, 456, 1973; Naoko Arai, *Gene introduction and Expression/Analytical Method (Idensi Donyu to Hatugen/Kaisekiho*), p. 13-15, 1994). After culturing for 30 hours, the culture medium was removed. The resulting cell was washed with a phosphate buffer (abbreviated as PBS hereinafter) and lysed with a lysis solution (100 mM potassium phosphate, pH 7.8, 0.2% Triton X-100) at 0.1 ml/well.

(2) Detection of human CbAP40 protein

10 μl of 2 × SDS sample buffer (125 mM Tris-HCl, pH 6.8, 3% sodium lauryl sulfate, 20% glycerin, 0.14 M β-mercaptoethanol, 0.02% bromophenol blue) was added to 10 μl of the lysate of the human CbAP40 expressing cell. After 2-min treatment at 100°C, the resulting lysate was subjected to 10% SDS polyacrylamide gel electrophoresis to separate the protein contained in the sample. Using a semi-dry type blotting apparatus (BioRad), the protein in the polyacrylamide was transferred onto a nitrocellulose membrane for detecting the human CbAP40 protein on the nitrocellulose by Western blotting according to the ordinary method. As a first antibody, a monoclonal antibody recognizing the V5 epitope fused with the C terminus of CbAP40 was used (Invitrogen), while as a second antibody, rabbit IgG-HRP fusion antibody (BioRad) was used. As shown in Fig. 1, consequently, it was confirmed that a protein of about 45 kDa representing the CbAP40-V5-His6 fusion protein consisting of 411 amino acids in total containing a tag of 45 amino acids at the C terminus was detected depending on the introduction of the expression vector pcDNA-CbAP40 into the cell. This result indicates that the full length human CbAP40 gene cloned into the culture cell was apparently expressed to possibly take a stable structure as a protein.

Example 3: Preparation of human CbAP40 protein

In order to insert the cDNA of human CbAP40 in a GST-fused expression vector pGEX-6P-1 (Amersham BioSciences), PCR was carried out using the primers represented by SEQ ID NOs:33 and 34 and pCDNA-CbAP40 prepared in Example 1 as a template to prepare a DNA fragment having a restriction *Bam*HI site and a restriction *Xho*I site added to the 5' and 3' ends, respectively, of the cDNA of the CbAP40 gene. Using DNA polymerase (Pyrobest DNA Polymerase; Takara Shuzo), PCR was carried out at 98°C (for 1 minute) and then by repeating a cycle of 98°C (for 5 seconds), 55°C (for 30 seconds) and 72°C (for 5 minutes) 35 times. The DNA fragment was treated enzymatically by *Bam*HI and *Xho*I to recombine the resulting fragment between the *Bam*HI and *Xho*I sites of pGEX-6P-1 to thereby prepare an expression plasmid pGEX-CbAP40.

Using pGEX-CbAP40, transformation of *E. coli* BL21 by heat shock was carried out. The resulting transformant cell was cultured overnight in 2.4 ml of a culture broth under agitation. Subsequently, the whole volume was transferred into 400 ml of a culture broth for culturing under agitation at 37°C for another 3 hours. Then, IPTG (Sigma) was added to give a final concentration of 2.5 mM for another 3-hour culturing under agitation to induce the expression of GST-fused CbAP40 protein (abbreviated as GST-CbAP40 hereinbelow). The bacterial cells were recovered, from which GST-CbAP40 was purified on glutathione Sepharose bead (Glutathione Sepharose 4B; Amersham Pharmacia) according to *Experimental Engineering* (*Jikken Kogaku*), Vol. 13, No. 6, 1994, p.528, Matsushime, *et al.*. As a control, the expression of a protein consisting of the GST part alone (abbreviated as GST protein hereinbelow) was induced in the *E. coli* BL21 transformed with pGEX-6P-1 in the same manner as described above. Then, the resulting GST protein was purified. Such purified proteins were separated by SDS gel electrophoresis by known methods and subsequent staining with Coomassie-blue. It was confirmed that proteins of the molecular weights as expected (GST-CbAP40: 67 kDa; GST protein: 26 kDa) were purified.

The purified sample of the CbAP40 protein can be used for various applications such as the analysis of interaction with c-Cbl and the preparation of antibodies against the CbAP40 protein. Specifically, the presence or absence of direct interaction with c-Cbl protein can be confirmed by the GST-pull down method (*Experimental Engineering (Jikken Kogaku*), Vol. 13, No. 6, 1994, p.528, Matsushime, *et al.*) according to the method described below in Example 9(3). More specifically, c-Cbl protein with a radioactive label can be prepared by *in vitro* transcription and translation using the cDNA of c-Cbl as a template and a TNT kit (TNT$^R$ Quick Coupled Transcription/Translation System; Promega) and a radioisotope (redivue Pro-mix L-[$^{35}$S]; Amersham) according to the attached protocol. After adding the GST-CbAP40 protein purified on the glutathione beads to the c-Cbl protein and subsequently shaking the resulting mixture at 4°C for one hour, a protein binding to the GST-CbAP40 protein on the beads is co-precipitated by centrifugation. The protein in the precipitate is separated by SDS polyacrylamide gel electrophoresis by known methods. Then, the labeled c-Cbl is detected by autoradiography to examine the direct interaction between the CbAP40 of the present invention and the c-Cbl protein.

Example 4: Analysis of tissue distribution of human CbAP40 gene expression

Using the primers represented by SEQ ID NOs:10 and 12, the full length cDNA fragment of the CbAP40 gene was amplified from human various tissues-derived cDNA by PCR to examine the presence or absence of the expression of CbAP40 in various tissues. PCR was carried out using 2 μg each of cDNA libraries derived from human bone marrow, brain, cartilage, heart, kidney, leukocyte, liver, lung, lymphocyte, mammary gland, ovary, pancreas, placenta, prostate, skeletal muscle, adipose, and artery as template and DNA polymerase (Pyrobest DNA Polymerase; Takara Shuzo, Co., Ltd.) at 98°C (for 1 minute) and by repeating a cycle of 98°C (for 5 seconds), 55°C (for 30 seconds) and 72°C (for 5 minutes) 35 times. The resulting PCR product was separated by agarose gel electrophoresis. A DNA fragment of about 1,100 base pairs considered to be desired human CbAP40 gene was amplified from the cDNA libraries derived from skeletal muscle and pancreas. These DNA fragments were separated from agarose gel to determine the nucleotide sequence of the DNA fragment using the primers represented by SEQ ID NO:12 according to the method described above in Example 1(4). Consequently, the DNA fragment was confirmed to be the human CbAP40 gene represented by SEQ ID NO:1. This result indicated that the expression of human CbAP40 gene was specifically regulated in very limited organs such as muscle and pancreas responding to insulin signaling.

Example 5: Measuring CbAP40 expression level in normal mice and diabetic model mice

Based on the findings above, it was demonstrated that the human CbAP40 protein of the present invention bound to c-Cbl was expressed in insulin responsive tissues such as skeletal muscle. Since the c-Cbl protein is a factor reacting with the second insulin signaling pathway, it was anticipated that the action of CbAP40 of the present invention was involved in insulin resistance. Therefore, the expression level of the messenger RNA (mRNA) of the CbAP40 gene was assayed in the muscles of normal mice C57BL/6J and m+/m+ and those of type 2 diabetic model mice KKA$^y$/Ta and db/db.

As the expression level of the gene, the expression level of the mouse CbAP40 gene of the present invention was measured. Then, the expression level of glyceraldehyde 3-phosphate dehydrogenase (G3PDH) gene was simultaneously measured and used for correcting the expression level of the mouse CbAP40 gene. As measurement systems, PRISM™ 7700 Sequence Detection System and SYBR Green PCR Master Mix (Applied BioSystems) were used. In the systems, the fluorescence of SYBR Green I dye incorporated into the double-stranded DNA amplified by PCR is detected and measured on real time to determine the expression level of the intended gene.

Specifically, the expression level of the gene was assayed by the following procedures.

(1) Preparation of total RNA

Male 15 week-old C57BL/6J, KKA$^y$/Ta, m+/m+ and db/db mice (all from CLEA JAPAN, INC.) were used. Muscle was resected from each mouse to prepare total RNA using an RNA extraction reagent (Isogen; Nippon Gene) according to the instruction thereof. The prepared total RNA each was thereafter treated by deoxyribonuclease (Nippon Gene), followed by phenol/chloroform treatment and ethanol precipitation. The resulting RNA was dissolved in distilled water and stored at -20°C.

(2) Synthesis of single-stranded cDNA

Reverse transcription of total RNA to single-stranded cDNA was carried out using 1 μg each of RNA prepared in (1) and a kit for reverse transcription (Advantage™ RT-for-PCR kit; Clontech) in a 20-μl system. After reverse transcription, 180 μl of distilled water was added for storage at -20°C.

(3) Preparation of PCR primer

Four oligonucleotides (SEQ ID NOs:13 to 16) were prepared as the primers for PCR described in (4). A combination of SEQ ID NOs:13 and 14 was used for mouse CbAP40 gene, while a combination of SEQ ID NOs:15 and 16 was used for G3PDH gene.

(4) Measuring gene expression level

PCR amplification was carried out in a 25-μl system on real time with RPISM™ 7700 Sequence Detection System according to the instruction. In each system, 5 μl of single-stranded cDNA, 12.5 μl of 2 × SYBR Green reagent and 7.5 pmol of each primer were used. Herein, the single-stranded cDNA stored in (2) was diluted 100-fold for use. For standard curve preparation, 0.1 μg/μl mouse genome DNA (Clontech) in place of the single-stranded cDNA was appropriately diluted, and 5 μl of the resulting dilution was used. PCR was carried out at 50°C for 10 minutes and continuously at 95°C for 10 minutes and then by repeating a cycle of two steps of 95°C for 15 seconds and 60°C for 60 seconds 45 times.

The expression level of the mouse CbAP40 gene in each sample was corrected on the basis of the expression level of the G3PDH gene according to the following equation:.

$$\text{Corrected CbAP40 expression level} = \text{Expression level of CbAP40 gene (raw data)} / \text{Expression level of G3PDH gene (raw data)}$$

For comparison of the expression level in muscle tissue, the expression level in C57BL/6J mouse was defined as 1 to express the relative levels as shown in Fig. 2. The values in the figure are expressed as mean ± SE. In the figure, the symbol * represents the significance $p < 0.05$ according to the assessment by the Dunnett's test.

As shown in Fig. 2, apparently, the expression of the mouse CbAP40 gene in accordance with the present invention was increased significantly in the muscle of the diabetic model mice. In humans, it is known that 75% of the glucose incorporation into cells depending on insulin is carried out in skeletal muscle. Thus, it is considered that CbAP40 of the present invention triggers insulin resistance by the elevation of the expression thereof in muscle. Thus, it is concluded that CbAP40 is deeply involved in insulin resistance.

The results of this Example indicate that the diagnosis of diabetic conditions can be established by assaying the CbAP40 expression level.

Example 6: Assaying glucose incorporation potency in cell highly expressing human CbAP40

(1) Preparation of virus highly expressing human CbAP40 using adenovirus vector

Such virus was prepared essentially on the basis of the following web site information (He T-C, *et al.*, A simplified system for rapid generation of recombinant adenoviruses. A practical guide for using the AdEasy system).

http://www.coloncancer.org/adeasy/protocol.htm

A fragment of the human CbAP40 gene was cleaved out of the pcDNA-CbAP40 prepared in Example 1 using restriction enzymes *Kpn*I and *Not*I. Using the same restriction enzymes, the human CbAP40 gene was subcloned into a vector pAdTrack-CMV (HeT-C., *et al.*, *Proc. Natl. Acad. Sci. USA*, 95, 2509-2514, 1998). The product was digested with a restriction enzyme *Pme*I, and then recombined in an adenovirus vector pAdEasy-1 in *E. coli.* The occurrence of the recombination was confirmed on the basis of a gene fragment of 4.5 kb as observed by digestion with a restriction enzyme *Pac*I and agarose gel electrophoresis. The recombinant virus vector was prepared and digested with a reaction enzyme *Pac*I for preparing a single strand, which was then introduced into a 293 cell using a lipofectamine 2000 reagent (Invitrogen). The virus highly expressing human CbAP40 was proliferated at a mass scale in the 293 cell and subsequently purified by density gradient centrifugation using cesium chloride as shown below for use in experiments.

First, the 293 cell infected with the virus highly expressing human CbAP40 was scraped from a petri dish coated with collagen, using a scraper and then collected by centrifugation at 1,500 rpm for 5 minutes. After removing the culture medium, the 293 cell was suspended in PBS, and then, was treated by repeating a process including three steps of freezing with dry ice ethanol, thawing in a warm bath at 37°C and vigorous suspension four times. In the procedures, the virus proliferating in the cell is released extracellularly. The cell suspension is centrifuged at 1,500 rpm for 5 minutes to collect the supernatant fraction. Then, a solution containing 43.9 g of NaCl, 3.7 g of KCl, 30.3 g of Tris and 1.42 g of $Na_2PO_4$ per one liter was adjusted to pH 7.4 using HCl. Cesium chloride was dissolved in the solution to prepare three kinds of cesium chloride solutions with densities of 1.339, 1.368 and 1.377. The cesium chloride solution with a density of 1.339 was overlaid on the cesium chloride solution with a density of 1.377, on which the virus supernatant fraction collected previously was additionally overlaid. Then, the resulting solution was ultra-centrifuged at 35,000 rpm for 1.5 hours using SW41 rotor manufactured by Beckman. Because the band observed at the lowest layer contained the virus, the layer was recovered with an 18-gauge syringe. The virus fraction was overlaid on the cesium chloride solution with a density of 1.368 and again ultra-centrifuged at 35,000 rpm for 18 hours. The virus was recovered with a 18-gauge syringe, transferred into a transparent tube and dialyzed against a dialysis solution (10 mM Tris-HCl, 1 mM $MgCl_2$, 135 mM NaCl, pH 7.5). After dialysis, the absorbance at 260 nm (A260) was measured to estimate the amount of the virus. The resulting value was corrected by the following equation. Glycerol was added to the virus fraction to 10%. Then, the virus was stored at -80°C until experimental use.
Equation:

$$1\ \text{A260} = 1.1 \times 10^{12}\ \text{virus particles} = 3.3 \times 10^{11}\ \text{pfu/ml}$$

(2) Differentiation into muscle cell and addition of human CbAP40 expressing adenovirus

Using L6 cell, the effect thereof on the glucose incorporation of CbAP40 was examined. L6 cell was suspended in $\alpha$-minimum essential culture medium containing 10% fetal calf serum (FCS) ($\alpha$MEM, Invitrogen) and then inoculated in a 24-well plate coated with collagen (Asahi Technoglass) to $1.6 \times 10^5$ cells/well. On the next day, the culture medium was exchanged with $\alpha$MEM containing 2% FCS for inducing the differentiation of the L6 cell into muscle. Three days thereafter, the culture medium was exchanged with 400 $\mu$l of the same culture medium. On the next day, human CbAP40 expressing adenovirus was added to the culture medium at a concentration of $1.6 \times 10^{10}$ pfu per well. As a control, adenovirus expressing eGFP alone was used.

(3) Measuring glucose incorporation potency in cell highly expressing human CbAP40

Twenty-four hours after the addition of adenovirus, the effect on glucose incorporation was evaluated. First, the culture medium was exchanged with 0.25 ml of KRP buffer (136 mM NaCl, 4.7 mM KCl, 1.25 mM $CaCl_2$, 1.25 mM $MgSO_4$, 5 mM $Na_2HPO_4$, pH 7.4) containing a predetermined concentration of insulin for incubation at 37°C for 20 minutes. Then, 15 $\mu$l of 2-deoxy-D-[U-$^{14}$C]glucose (Amersham BioSciences) was added to 1 ml of KRP containing 1 mM 2-deoxy-D-glucose. Next, 50 $\mu$l each of the resulting buffer was added to each well for incubation at 37 °C for 10 minutes. Thereafter, washing three times with a cooled phosphate buffered physiological solution (PBS) was carried out. The cell was lysed with 0.1% sodium lauryl sulfate (SDS) and mixed with 2 ml of a scintillator (Aquazol-2, Packard BioSciences) to measure the glucose incorporated in the cell by a liquid scintillation counter (Tricurb B2500TR, Packard). The results are shown in Fig. 3. The numerical values in the figure are expressed as mean $\pm$ SE. According to the assessment by the Dunnett's test, the symbol * represents significance $p < 0.05$. The symbol ** represents significance $p < 0.01$.

As shown in Fig. 3, it was found that glucose incorporation was decreased when the human CbAP40 gene was highly expressed in muscle cell.

Example 7: Identification of promoter sequence of human CbAP40 gene and screening system of compound for improving insulin resistance, utilizing transcription induction activity of the sequence

(1) Cloning the promoter of human CbAP40 gene

It is known that the expression level of CAP (Cbl-associated protein) reported as a molecule binding to c-Cbl on the second insulin signaling pathway is increased with thiazolidine derivatives as agents for improving insulin resistance. It is considered that the increase of the expression level of CAP is more or less involved in the action of thiazolidine derivatives to improve insulin resistance. Like CAP, the CbAP40 of the present invention binds to c-Cbl. Based on the facts described above, CbAP40 is considered to be an exacerbation factor of diabetes in contrast to CAP, because CbAP40 causes insulin resistance. Therefore, it was speculated that CbAP40 expression could be regulated in a manner in contrast to that of CAP. However, no promoter sequence involved in the expression and regulation of human CbAP40 was clearly identified yet. Therefore, the human CbAP40 promoter sequence was obtained to arrange a reporter gene in the downstream of the promoter sequence to construct an assayable system by detecting the expression of human CbAP40 so as to examine the regulation mechanism of the human CbAP40 gene expression.

A pair of primers represented by SEQ ID NOs:17 and 18 were designed. Using these primers, a polynucleotide including the promoter sequence of human CbAP40 was amplified by PCR, using DNA polymerase (LA Taq DNA polymerase; Takara Shuzo) using human genome DNA (Clontech) as a template. PCR reaction conditions were as follows: 98°C (for 5 minutes), and a cycle of 96°C (for 30 seconds), 55°C (for 30 seconds) and 72°C (for 90 seconds) as repeated 35 times. Subsequently, the resulting solution was heated at 72°C for 7 minutes. Consequently, a polynucleotide of about 3.1 kbp was successfully amplified. In order to demonstrate that the polynucleotide contained the promoter regulating the expression of human CbAP40, the DNA fragment obtained by the PCR was treated with restriction enzymes *Xho*I and *Bam*HI (Takara Shuzo) and then conjugated to luciferase reporter vector (pGL3-Basic vector; Promega) to construct CbAP40 gene promoter-conjugated reporter vector (pGL3-CbAP40p).

The nucleotide sequence of the 3.1-kb polynucleotide inserted into pGLC3-CbAP40p was partially determined, using primers represented by SEQ ID NOs:17 and 18 and DNA primers represented by SEQ ID NOs:19 and 20 (Proligo) binding to the two ends of the multi-cloning site of the pGLC-Basic vector. Using additional four types of DNA primers represented by SEQ ID NOs:21, 22, 23 and 24, as designed on the basis of the determined nucleotide sequence information, the full length nucleotide sequence of the polynucleotide was determined. Consequently, it was found that the polynucleotide was the 3119-bp polynucleotide represented by SEQ ID NO:3.

Based on the nucleotide sequence information, further, pGL3-CbAP40p was digested with restriction enzyme *Hin*dIII and ligated to the plasmid by a ligation reaction to prepare a plasmid pGL3-CbAP40p[1-1231] containing the polynucleotide represented by SEQ ID NO:3 from which the nucleotides at positions 1231 to 3119 were removed. Furthermore, pGL3-CbAP40p was digested with restriction enzymes *Sma*I and *Hin*dIII to scissor out a DNA fragment corresponding to a region of positions 1364 to 3119 in the polynucleotide represented by SEQ ID NO: 3, which was then ligated to the pGL3-Basic vector digested with restriction enzymes *Sma*I and *Hin*dIII to prepare pGL3-CbAP40p [1364-3119]. Using two pairs of primers, i.e. a primer pair represented by SEQ ID NOs:18 and 23 and a primer pair represented by SEQ ID NOs:18 and 24, further, PCR under the same conditions as in Example 7(1) was carried out using pGL3-CbAP40p as a template to individually extract a DNA fragment of positions 2125 to 3119 in the polynucleotide represented by SEQ ID NO:3 and a DNA fragment of the nucleotides at positions 2569 to 3119. These DNA fragments were individually digested with restriction enzymes *Sac*I and *Bam*HI and ligated to pGL3-Basic vector digested with restriction enzymes *Sac*I and *Bgl*II in the same manner to respectively prepare pGL3-CbAP40p[2125-3119] and pGL3-CbAP40p[2569-3119]. The nucleotide sequences of the inserted sequences in these constructs pGL3-CbAP40p[1-1231], pGL3-CbAP40p[1364-3119], pGL3-CbAP40p[2125-3119] and pGL3-CbAP40p[2569-3119] were all determined using the DNA primers represented by SEQ ID NOs:19 and 20. Consequently, all the constructs contained partial sequences of the polynucleotide represented by SEQ ID NO:3. It was confirmed that the constructed plasmids respectively contained the regions of the nucleotide sequences of the polynucleotide, which correspond to the numerical figures expressed in parenthesis in each plasmid name.

(2) Construction of screening system of compound utilizing transcription induction activity of human CbAP40 promoter

According to the method described in Example 2(1), pGL3-CbAP40p was transfected into Cos-1 cells. Compared with the case of transfection with the vacant vector pGL3-Basic vector, the expression induction activity of the polynucleotide as the promoter was assayed using the activity of luciferase as an indicator. The correction of the transfection efficiency into cells and luciferase assay were carried out by the following methods described in detail below. A culture cell, namely 293 cell (Cell Bank) was cultured in a 12-well culture plate (well diameter of 22 mm) until 70% confluence, where the minimum essential culture medium DMEM (Gibco) containing 10% fetal calf serum (Sigma) was added at 1 ml per well. The cell was transiently transfected with pGL3-CbAP40p or pGL3-Basic Vector (0.8 μg/well) according to the attached protocol, using lipofectamine method (LIPOFECTAMINE™ 2000; Invitrogen). Pioglitazone [(+)-5-[4-[2-(5-ethyl-2-pyridienyl)ethoxy]benzyl]-2,4-thiazolidinone] was added at 0.1 μM, 1.0 μM or 10 μM to the culture medium for 24-hour culturing., The culture medium was removed and the cell was washed with PBS. Then, 0.1 ml of a cell lysis solution (100 mM potassium phosphate, pH 7.8, 0.2% Triton X-100) was added per well for cell lysis. Pioglitazone was synthesized by the method described in the specification of Japanese Patent No. 1853588.

100 μl of a luciferase substrate solution (Picker gene) was added to 100 μl of the cell lysate to measure chemiluminescent counts per 10 seconds using a chemiluminescence counter of Type AB-2100 (Atto Corporation). The cell was transfected with a plasmid pCH110 (Amersham Pharmacia Biotech) containing the luciferase reporter gene together with the β-galactosidase expressing gene at 0.1 μl/well to measure and numerically express the β-galactosidase activity using a Galacto-Light Plus™ kit system (Applied Biosystems) for detecting β-galactosidase activity. Using the resulting numerical value as the transfection efficiency of the introduced gene, the luciferase activity of each well obtained above was corrected.

The results are shown in Fig. 4. The values in the drawing are expressed as mean ± SE. A significant promoter activity depending on the sequence upstream the human CbAP40 gene was confirmed. It was demonstrated that the promoter activity was suppressed by pioglitazone, an agent for improving insulin resistance and one of thiazolidine derivatives,, when added at 0.1 to 10 μM. Furthermore, the same experiments were carried out using pGL3-CbAP40p [1-1231], pGL3-CbAP40p[1364-3119], pGL3-CbAP40p[2125-3119] and pGL3-CbAP40p[2569-3119] in place of pGL3-CbAP40p. When pGL3-CbAP40p[1364-3119] or pGL3-CbAP40p[2125-3119] was used, the promoter activity was detected and suppressed by pioglitazone. When pGL3-CbAP40p[2569-3119] was used, the promoter activity was detected but never suppressed effectively by pioglitazone under observation. When pGL3-CbAP40p[1-1231] was used, the promoter activity was not detected. Thus, it is indicated that the nucleotide sequence of positions 2125 to 3119 in the polynucleotide represented by SEQ ID NO:3 was satisfactorily contained for the expression of the promoter activity. Additionally, the pioglitazone action on the suppression of the promoter activity was apparently induced depending on the presence of the DNA sequence of the nucleotides at positions 2125 to 2569 in the polynucleotide represented by SEQ ID NO:3. In other words, the polynucleotide of the nucleotide sequence represented by SEQ ID NO:3 and the polynucleotides of the nucleotide sequences represented by positions 1364 to 3119 and positions 2125 to 3119 in the nucleotide sequence represented by SEQ ID NO:3 contained a promoter sequence regulating the expression of human CbAP40 and that the promoter was downregulated with PPARγ ligands such as pioglitazone decreasing insulin resistance. Based on the fact, it was speculated that insulin resistance was dropped by the suppression of CbAP40 expression with thiazolidine derivatives decreasing insulin resistance.

Accordingly, the promoter assay of human CbAP40 in this Example can be utilized for screening of PPARγ ligands or agents for improving insulin resistance without using PPARγ protein or the response sequence thereof.

Since the pioglitazone action of suppressing the promoter activity is induced depending on the presence of the nucleotide sequence at positions 2125 to 2569 in the nucleotide sequence represented by SEQ ID NO:3, further, a polynucleotide containing the sequence part is arranged upstream a promoter sequence of a gene containing TATA box required for transcription induction in the minimum length except CbAP40, so that the polynucleotide can be utilized for screening of PPARγ ligands or agents for improving insulin resistance without using the PPARγ protein or the response sequence thereof alike.

The compounds obtained by the screening method include those with structural features different from those of typical PPARγ ligands such as thiazolidine derivatives obtained via the conventional method using PPARγ protein. In other words, an agent for improving type 2 diabetes with no side effects such as edema and the increase of fat weight as observed for thiazolidine derivatives can be obtained.

Example 8: Cloning of mouse CbAP40

(1) Cloning of mouse CbAP40

Using a single-stranded DNA library based on the template mRNA derived from the muscle of a diabetic model mouse as prepared in Example 5 as a template, PCR was carried out by a known method to prepare a cDNA library of double-stranded DNA. Using the DNA as a template and a pair of primers represented by SEQ ID NOs:27 and 28, the same PCR as in Example 1(3) was carried out to amplify the full length cDNA of the orthologous gene of CbAP40 mouse. The nucleotide sequence of the resulting DNA fragment of about 1.4 kbp was determined. It was confirmed that the DNA fragment contained the full length cDNA of the 1404-bp gene represented by SEQ ID NO:25. The cDNA is a novel gene encoding the polypeptide represented by SEQ ID NO:26. Although the known genes NM_172708 and AK044445 registered on GenBank partially contain the same sequence as that of the novel gene, the 3' terminal cDNAs are different. Thus, the polypeptides encoded thereby are totally different in view of carboxyl terminal length and sequence. In contrast, the novel gene has a C terminal structure almost identical to that of human CbAP40 and has 75.6% homology to the human CbAP40 gene represented by SEQ ID NO:1, while the polypeptide encoded thereby has 71.1% homology to the human CbAP40 protein represented by SEQ ID NO:2. Their homology levels are so high. The findings indicate that the novel gene is an orthologous gene of the human CbAP40 of the present invention. Accordingly, it can be said that the human CbAP40 has the same functions as those of the mouse CbAP40.

(2) Preparation of mouse CbAP40 expression vector

According to the same method as the method described in Example 1(4), the mouse CbAP40 cDNA was cloned into pcDNA3.1-VS-TOPO (Invitrogen). In order to eliminate the stop codon of mouse CbAP40 for tag fusion, primers represented by SEQ ID NOs:29 and 27 were used for PCR and recloning into a vector. The prepared expression vector was named pcDNA-mCbAP40.

(3) Preparation of mouse CbAP40 expressing cell and detection of mouse CbAP40 protein

According to the method described in Example 2(1), pcDNA-mCbAP40 was transiently introduced into the 293 cell using calcium phosphate method. After culturing for 30 hours, the culture medium was removed. The resulting cell was washed with PBS and lysed with 0.1 ml of cell lysis solution (100 mM calcium phosphate, pH 7.8, 0.2% Triton X-100) per well. Continuously, the mouse CbAP40 protein was detected according to the method described in Example 2(2), using separation by polyacrylamide gel electrophoresis and Western blotting using anti-V5 antibody. Consequently, it was confirmed that a protein of about 60 kDa was detected, depending on the introduction of the expression vector pcDNA-mCbAP40. The detected protein was a mouse CbAP40-V5-His6 fusion protein of 512 amino acid residues in total, containing a C terminal tag of 45 amino acid residues. This indicates that the full length gene of the mouse CbAP40 cloned into the culture cell was certainly expressed, so that the resulting protein was in a stable structure.

Example 9: Demonstration of interactions of human and mouse CbAP40 with c-Cbl

(1) Preparation of GST-fused c-Cbl expression plasmid

In order to insert cDNA of mouse c-Cbl into the GST-fused expression vector pGEX-6P-1 (Amersham Bioscience), PCR using the cDNA of mouse c-Cbl as obtained in Example 1(1) as a template and DNA oligoprimers (Proligo) represented by SEQ ID NOs:30 and 31 was carried out to add individually restriction enzyme sites of *Eco*RV site and *Xho*I site to the two ends of the cDNA. Herein, the PCR was carried out under the conditions described in Example 1 (1). The cDNA fragment was cleaved with restriction enzymes *Eco*RV and *Xho*I, while the vector pGEX-6P-1 was cleaved with restriction enzymes SmaI and *Xho*I into a linear chain. The two cleaved products were mixed together and combined with a DNA ligase solution (DNA ligation kit II; Takara Shuzo) for treatment at 16°C for 3 hours to prepare a plasmid (abbreviated as pGEX-Cbl hereinafter) with the c-Cbl cDNA inserted in the multicloning site of pGEX-6P-1. Using the oligonucleotide represented by SEQ ID NO:32 as primer and a sequencing kit (Applied BioSystems) and a sequencer (ABI 3700 DNA Sequencer of Applied BioSystems), the nucleotide sequence was determined to select a plasmid where the cDNA coding region of c-Cbl and the GST tag translation frame of the pGEX vector were inserted together in the same frame.

(2) Purification of GST-fused c-Cbl protein

Using the plasmid pGEX-Cbl obtained above in (1), GST-Cbl was purified in the same manner as in Example 3. As a control, the expression of a protein consisting of the GST part alone (abbreviated as GST protein hereinafter) was induced in *E. coli* BL21 transformed with pGEX-6P-1 in the same manner as described above. Then, the resulting protein was purified. According to known methods, separation by SDS polyacrylamide gel electrophoresis and staining with Coomassie Brilliant Blue were carried out to confirm that the protein of the desired molecular weight (GST-Cbl: 100 kDa; GST protein: 26 kDa) was obtained.

(3) Confirmation of biological association between c-Cbl protein and human or mouse CbAP40 protein

Using the protein GST-Cbl prepared above in (2), the presence or absence of the direct interaction of human and mouse CbAP40 proteins with the c-Cbl protein was confirmed by the GST-pull down method *(Zikken Kougaku*, Vol. 13, No. 6, 1994, p. 528, Matsushime, *et al.*). Using 0.5 μg of pcDNA-CbAP40 prepared above in Example 1(4) or pcDNA-mCbAP40 prepared above in Example 8(2) as a template, and additionally using 40 μl of TNT kit (TNT$^R$ Quick Coupled Transcription/Translation System; Promega) and 1.3 MBq of a radioisotope (redivue Pro-mix L-[$^{35}$S]; Amersham) according to the attached protocol, human or mouse CbAP40 protein radioactively labeled was prepared by *in vitro* transcription and translation. Then, 15 μl each of the prepared solution of human or mouse CbAP40 protein was mixed with 1 μl of the GST protein or GST-Cbl purified on glutathione beads as described above in (2) to which 0.3 ml of Buffer A (50 mM Tris-HCl, pH 7.5, 10% glycerol, 120 mM NaCl, 1 mM EDTA, 0.1 mM EGTA, 0.5 mM PMSF, 0.5% NP-40) was added. The resulting mixture was shaken at 4°C for one hour. Subsequently, the protein binding to the GST protein or GST-Cbl on the bead was co-precipitated by centrifugation. This co-precipitate was suspended in 0.5 ml of a buffer prepared by replacing the Buffer A with 100 mM NaCl, and was co-precipitated again by centrifugation. After the procedure was repeatedly carried out four times, the protein in the precipitate was separated by SDS polyacrylamide gel electrophoresis by known methods to detect the human or mouse CbAP40 protein by autoradiography. Consequently, a band never detected in mixing the GST protein was detected in case of mixing GST-Cbl. This result apparently indicates that human or mouse CbAP40 as one type of the polypeptide of the present invention similarly interact with the c-Cbl protein, supporting that these human and mouse CbAP40s are counterparts having the same functions in the two animal species. Thus, it is found that the mouse CbAP40 of the present invention is involved in triggering insulin resistance by the interaction with c-Cbl protein, like the human CbAP40 of the present invention.

Industrial Applicability

CbAP40 is a novel molecule involved in insulin signaling. The polypeptide, the polynucleotide, the expression vector and the cell of the present invention are useful for identifying and screening for an agent for improving type 2 diabetes, particularly an agent for improving insulin resistance or an agent for improving glucose metabolism. According to the screening method of the present invention, screening for an agent for improving type 2 diabetes can be carried out. Additionally, the polypeptide of the present invention and the polynucleotide encoding the polypeptide of the present invention are useful for the diagnosis of diabetes.

Sequence Listing Free Text

In the numerical title <223> in the Sequence Listing below, the Artificial Sequence is described. Specifically, respective nucleotide sequences represented by SEQ ID NOs:8, 9, 11, 19, 20, 30, 31, 33 and 34 in the Sequence Listing are primer sequences artificially synthesized.

While the invention has been described with reference to specific embodiments thereof, changes and modifications obvious for one skilled in the art are within the scope of the invention.

## Claims

**1.** A method for assaying whether or not a test substance is capable of inhibiting promoter activity of a polynucleotide of any one of the following (i) to (iv), which comprises:

(1) a step of bringing a test substance into contact with a cell transformed with an expression vector containing a polynucleotide which consists of (i) the nucleotide sequence represented by SEQ ID NO:3, (ii) the nucleotide sequence represented by positions 1364 to 3119 in the nucleotide sequence represented by SEQ ID NO:3, or (iii) the nucleotide sequence represented by positions 2125 to 3119 in the nucleotide sequence represented by SEQ ID NO:3; or a polynucleotide which comprises (iv) a nucleotide sequence in which 1 to 10 nucleotides are deleted, substituted and/or inserted in any one of the nucleotide sequences represented by (i) to (iii), and which has promoter activity of a polypeptide consisting of the amino acid sequence represented by SEQ ID NO:2 or SEQ ID NO:26; and
(2) a step of detecting the promoter activity.

**2.** A method for screening a substance capable of suppressing expression of the polypeptide according to claim 1, which comprises:

an analysis step by the method according to claim 1; and
a step of selecting a substance capable of inhibiting the promoter activity.

**3.** A method for screening an agent for improving type 2 diabetes by the method according to claim 2.

**4.** A polynucleotide consisting of (1) the nucleotide sequence represented by SEQ ID NO:3, (2) the nucleotide sequence represented by positions 1364 to 3119 in the nucleotide sequence represented by SEQ ID NO:3, or (3) the nucleotide sequence represented by positions 2125 to 3119 in the nucleotide sequence represented by SEQ ID NO:3; or a polynucleotide which consists of (4) a nucleotide sequence in which 1 to 10 nucleotides are deleted, substituted, inserted and/or added in any one of the nucleotide sequences represented by (1) to (3), and which has promoter activity of the polypeptide according to claim 1.

**5.** A method for assaying whether or not a test substance is capable of inhibiting binding of a polypeptide to c-Cbl, which comprises:

a step of bringing the polypeptide and c-Cbl into contact with a test substance, wherein the polypeptide comprises (1) the amino acid sequence represented by SEQ ID NO:2 or SEQ ID NO:26, (2) an amino acid sequence in which 1 to 10 amino acids are deleted, substituted and/or inserted in the amino acid sequence represented by SEQ ID NO:2 or SEQ ID NO:26, or (3) an amino acid sequence having 90% or more homology to the amino acid sequence represented by SEQ ID NO:2 or SEQ ID NO:26, and is capable of inhibiting glucose incorporation by binding to c-Cbl and/or overexpression; and
a step of detecting binding of the polypeptide to c-Cbl.

**6.** A method for screening a substance capable of inhibiting binding of the polypeptide according to claim 5 to c-Cbl, which comprises:

an assaying step by the method according to claim 5, and
a step of selecting a substance capable of inhibiting the binding.

**7.** A method for screening an agent for improving type 2 diabetes by the method described in claim 6.

**8.** A polypeptide which comprises the amino acid sequence represented by SEQ ID NO:2 or SEQ ID NO:26 or an amino acid sequence in which 1 to 10 amino acids are deleted, substituted and/or inserted in the amino acid sequence represented by SEQ ID NO:2 or SEQ ID NO:26, and which is capable of inhibiting glucose incorporation by binding to c-Cbl and/or overexpression.

**9.** A polypeptide consisting of the amino acid sequence represented by SEQ ID NO:2 or SEQ ID NO:26.

**10.** A polynucleotide encoding a polypeptide which consists of the amino acid sequence represented by SEQ ID NO: 26; or a polypeptide which consists of an amino acid sequence in which 1 to 10 amino acids are deleted, substituted, inserted and/or added in the amino acid sequence represented by SEQ ID NO:26, and which is capable of inhibiting glucose incorporation by binding to c-Cbl and/or overexpression.

**11.** An expression vector containing the polynucleotide according to claim 4 or 10.

**12.** A cell transformed with the expression vector according to claim 11.

**13.** A screening tool of an agent for improving type 2 diabetes, which comprises comprising (1) the polypeptide according to claim 8, (2) a polynucleotide encoding the polypeptide according to claim 8 or the polynucleotide of any one of (i) to (iv) according to claim 1, or (3) a polynucleotide encoding the polypeptide according to claim 8 or a cell transformed with an expression vector containing the polynucleotide of any one of (i) to (iv) according to claim 1.

**14.** Use of (1) the polypeptide according to claim 8, (2) a polynucleotide encoding the polypeptide according to claim 8 or the polynucleotide of any one of (i) to (iv) according to claim 1, or (3) a polynucleotide encoding the polypeptide according to claim 8 or a cell transformed with an expression vector containing the polynucleotide of any one of (i) to (iv) according to claim 1 for screening of an agent for improving type 2 diabetes.

FIG. 1

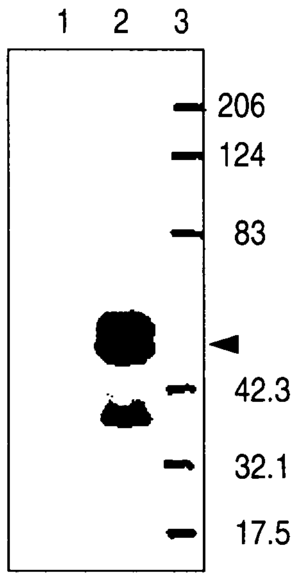

FIG. 2

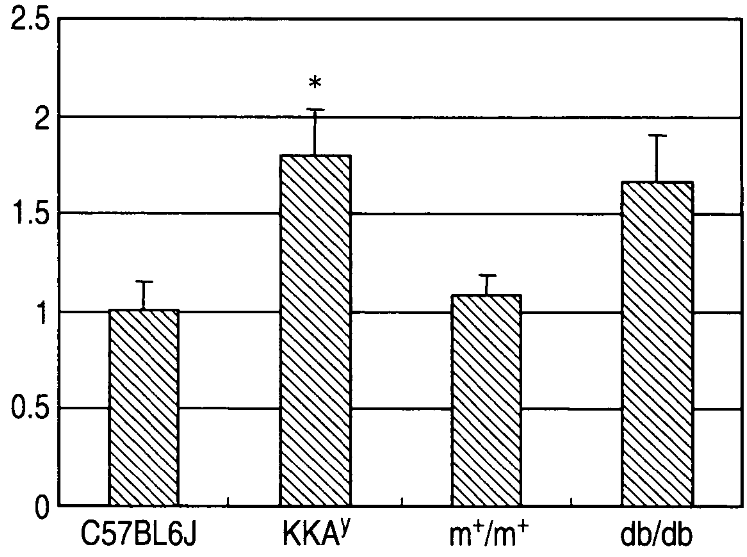

FIG. 3

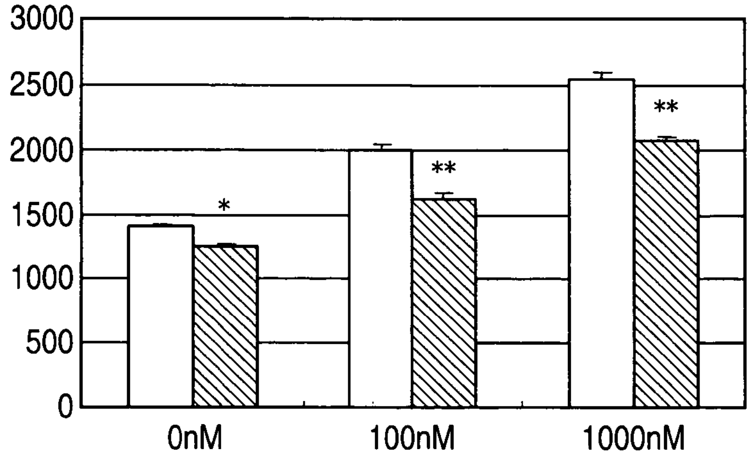

FIG. 4

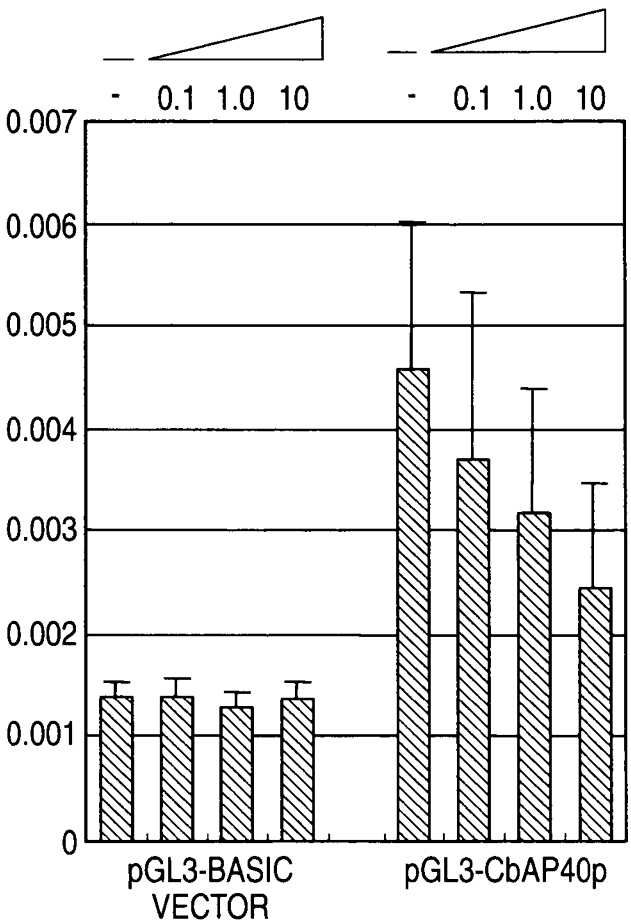
- 0.1 1.0 10
- 0.1 1.0 10
0.007
0.006
0.005
0.004
0.003
0.002
0.001
0
pGL3-BASIC VECTOR
pGL3-CbAP40p

**INTERNATIONAL SEARCH REPORT**

International application No.
PCT/JP2004/011585

A. CLASSIFICATION OF SUBJECT MATTER
Int.Cl[7] C12N15/11, C12Q1/68, C07K14/47, G01N33/50

According to International Patent Classification (IPC) or to both national classification and IPC

B. FIELDS SEARCHED

Minimum documentation searched (classification system followed by classification symbols)
Int.Cl[7] C12N15/11, C12Q1/68, C07K14/47, G01N33/50

Documentation searched other than minimum documentation to the extent that such documents are included in the fields searched

Electronic data base consulted during the international search (name of data base and, where practicable, search terms used)
GenBank/EMBL/DDBJ/Swissprot/PIR/GeneSeq,
WPIDS/BIOSIS/BIOTECHABS/MEDLINE/CA(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 2003-135075 A (Research Association for Biotechnology), 13 May, 2003 (13.05.03), Seq. Nos. 269, 2239 & US 2003/0236392 A1 | 4,8-12<br>1-3,5-7,13, 14 |
| A | Chiang S.H. et al., Cloning and functional characterization of related TC10 isoforms, a subfamily of Rho proteins involved in insulin-stimulated glucose transport, J.Biol. Chem., 2002, Vol.277, pages 13067 to 13073 | 1-14 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 October, 2004 (15.10.04) | 02 November, 2004 (02.11.04) |
| Name and mailing address of the ISA/<br>Japanese Patent Office | Authorized officer |
| Facsimile No. | Telephone No. |

Form PCT/ISA/210 (second sheet) (January 2004)

SEQUENCE LISTING

<110> Yamanouchi Pharmaceutical Co.,Ltd.

<120> A novel protein available for screening of antidiabetic agents

<130> Y0425-PCT

<150> JP2003-206948
<151> 2003-08-08

<150> JP2004-000732
<151> 2004-01-06

<160> 34

<170> PatentIn version 3.2

<210> 1
<211> 1101
<212> DNA
<213> Homo sapiens

<220>
<221> CDS
<222> (1)..(1098)
<223> Inventor: Endoh, Hideki; Ueda, Yoshitaka

<400> 1

```
atg atg tcc tcg tct tgg cca ggg aca tcc ccc cgg ctg tca cgg ggc       48
Met Met Ser Ser Ser Trp Pro Gly Thr Ser Pro Arg Leu Ser Arg Gly
1               5                   10                  15

agt gga agc tgt ctg acc tcc ggc gct acg ggg ccg tgc caa gcg gat       96
Ser Gly Ser Cys Leu Thr Ser Gly Ala Thr Gly Pro Cys Gln Ala Asp
            20                  25                  30

tca tct ttg aag gcg gga cca ggg gct ggc gtc ttc ttc ctg tcc tcg      144
Ser Ser Leu Lys Ala Gly Pro Gly Ala Gly Val Phe Phe Leu Ser Ser
        35                  40                  45

gcc gag ggg gag cag atc agc ttc ctg ttc gac tgc atc gtc cga ggc      192
Ala Glu Gly Glu Gln Ile Ser Phe Leu Phe Asp Cys Ile Val Arg Gly
    50                  55                  60
```

```
atc tcc ccc acc aag ggc ccc ttt ggg ctg cgg ccg gtt cta cca gac      240
Ile Ser Pro Thr Lys Gly Pro Phe Gly Leu Arg Pro Val Leu Pro Asp
65                  70                  75                  80

cca agt ccc ccg gga ccc tcg act gtg gag gag cgt gtg gcc cag gaa      288
Pro Ser Pro Pro Gly Pro Ser Thr Val Glu Glu Arg Val Ala Gln Glu
                85                  90                  95

gcc ctg gaa acc cta cag ctg gag aag cgg ctg agc ctc ctc tca cat      336
Ala Leu Glu Thr Leu Gln Leu Glu Lys Arg Leu Ser Leu Leu Ser His
            100                 105                 110

gcg ggc agg ccg ggc agt gga ggg gat gac cgc agc ctg tcc agc tca      384
Ala Gly Arg Pro Gly Ser Gly Gly Asp Asp Arg Ser Leu Ser Ser Ser
        115                 120                 125

tcc tca gag gcc agt cac ttg gac gtc agc gcc agc agc cgg ctc acc      432
Ser Ser Glu Ala Ser His Leu Asp Val Ser Ala Ser Ser Arg Leu Thr
    130                 135                 140

gca tgg cca gag caa tcc tcg tcg tca gcc agc acg tca cag gag ggg      480
Ala Trp Pro Glu Gln Ser Ser Ser Ser Ala Ser Thr Ser Gln Glu Gly
145                 150                 155                 160

cct aga cca gca gct gcc cag gcc gcc ggg gaa gcc atg gtg ggt gcc      528
Pro Arg Pro Ala Ala Ala Gln Ala Ala Gly Glu Ala Met Val Gly Ala
                165                 170                 175

tca agg cca ccc ccc aag ccg ctg cgt ccg cgg cag ctg cag gag gtt      576
Ser Arg Pro Pro Pro Lys Pro Leu Arg Pro Arg Gln Leu Gln Glu Val
            180                 185                 190

ggc cgc cag agc tcc tcg gac agc ggc atc gcc act ggc agc cac tcc      624
Gly Arg Gln Ser Ser Ser Asp Ser Gly Ile Ala Thr Gly Ser His Ser
        195                 200                 205

tct tac tcc agc agc ctc tcg tcc tac gcg ggc agc agc ctg gac gtg      672
Ser Tyr Ser Ser Ser Leu Ser Ser Tyr Ala Gly Ser Ser Leu Asp Val
    210                 215                 220

tgg cgg gcc aca gat gaa ctg ggc tca ctg ctc agc ctg cca gca gcg      720
Trp Arg Ala Thr Asp Glu Leu Gly Ser Leu Leu Ser Leu Pro Ala Ala
225                 230                 235                 240

ggg gcc ccc gag ccc agc ctg tgc acc tgc ctg ccc ggg aca gtc gag      768
Gly Ala Pro Glu Pro Ser Leu Cys Thr Cys Leu Pro Gly Thr Val Glu
```

245 250 255

tac cag gtg ccc acc tcc ctg cgg gcc cac tat gac aca cca cgc agc 816
Tyr Gln Val Pro Thr Ser Leu Arg Ala His Tyr Asp Thr Pro Arg Ser
260 265 270

ctt tgc ctg gct cct aga gac cac agc ccc ccc tca cag ggc agc ccc 864
Leu Cys Leu Ala Pro Arg Asp His Ser Pro Pro Ser Gln Gly Ser Pro
275 280 285

ggc aac agt gcg gcc agg gac tca ggc ggc cag acg tcc gcc ggg tgt 912
Gly Asn Ser Ala Ala Arg Asp Ser Gly Gly Gln Thr Ser Ala Gly Cys
290 295 300

ccc tct ggc tgg ctg ggc acg aga cgg cgg ggc ctg gtg atg gag gcc 960
Pro Ser Gly Trp Leu Gly Thr Arg Arg Arg Gly Leu Val Met Glu Ala
305 310 315 320

ccc cag ggc agc gag gcc aca ctg cct ggc cct gcc cct ggc gag ccc 1008
Pro Gln Gly Ser Glu Ala Thr Leu Pro Gly Pro Ala Pro Gly Glu Pro
325 330 335

tgg gaa gca ggc ggc ccc cac gcg ggg cca ccc ccg gct ttc ttt tcg 1056
Trp Glu Ala Gly Gly Pro His Ala Gly Pro Pro Pro Ala Phe Phe Ser
340 345 350

gca tgt cca gtc tgt gga gga ctc aag gta aac ccc cct cct tga 1101
Ala Cys Pro Val Cys Gly Gly Leu Lys Val Asn Pro Pro Pro
355 360 365

<210> 2
<211> 366
<212> PRT
<213> Homo sapiens

<400> 2

Met Met Ser Ser Ser Trp Pro Gly Thr Ser Pro Arg Leu Ser Arg Gly
1 5 10 15

Ser Gly Ser Cys Leu Thr Ser Gly Ala Thr Gly Pro Cys Gln Ala Asp
20 25 30

Ser Ser Leu Lys Ala Gly Pro Gly Ala Gly Val Phe Phe Leu Ser Ser
35 40 45

Ala Glu Gly Glu Gln Ile Ser Phe Leu Phe Asp Cys Ile Val Arg Gly
50 55 60

Ile Ser Pro Thr Lys Gly Pro Phe Gly Leu Arg Pro Val Leu Pro Asp
65 70 75 80

Pro Ser Pro Pro Gly Pro Ser Thr Val Glu Glu Arg Val Ala Gln Glu
85 90 95

Ala Leu Glu Thr Leu Gln Leu Glu Lys Arg Leu Ser Leu Leu Ser His
100 105 110

Ala Gly Arg Pro Gly Ser Gly Gly Asp Asp Arg Ser Leu Ser Ser Ser
115 120 125

Ser Ser Glu Ala Ser His Leu Asp Val Ser Ala Ser Ser Arg Leu Thr
130 135 140

Ala Trp Pro Glu Gln Ser Ser Ser Ser Ala Ser Thr Ser Gln Glu Gly
145 150 155 160

Pro Arg Pro Ala Ala Ala Gln Ala Ala Gly Glu Ala Met Val Gly Ala
165 170 175

Ser Arg Pro Pro Pro Lys Pro Leu Arg Pro Arg Gln Leu Gln Glu Val
180 185 190

Gly Arg Gln Ser Ser Ser Asp Ser Gly Ile Ala Thr Gly Ser His Ser
195 200 205

Ser Tyr Ser Ser Ser Leu Ser Ser Tyr Ala Gly Ser Ser Leu Asp Val
210 215 220

Trp Arg Ala Thr Asp Glu Leu Gly Ser Leu Leu Ser Leu Pro Ala Ala
225                 230                 235                 240

Gly Ala Pro Glu Pro Ser Leu Cys Thr Cys Leu Pro Gly Thr Val Glu
                245                 250                 255

Tyr Gln Val Pro Thr Ser Leu Arg Ala His Tyr Asp Thr Pro Arg Ser
            260                 265                 270

Leu Cys Leu Ala Pro Arg Asp His Ser Pro Pro Ser Gln Gly Ser Pro
        275                 280                 285

Gly Asn Ser Ala Ala Arg Asp Ser Gly Gly Gln Thr Ser Ala Gly Cys
    290                 295                 300

Pro Ser Gly Trp Leu Gly Thr Arg Arg Arg Gly Leu Val Met Glu Ala
305                 310                 315                 320

Pro Gln Gly Ser Glu Ala Thr Leu Pro Gly Pro Ala Pro Gly Glu Pro
                325                 330                 335

Trp Glu Ala Gly Gly Pro His Ala Gly Pro Pro Pro Ala Phe Phe Ser
            340                 345                 350

Ala Cys Pro Val Cys Gly Gly Leu Lys Val Asn Pro Pro Pro
        355                 360                 365

<210> 3
<211> 3119
<212> DNA
<213> Homo sapiens

<220>
<221> promoter

<222> (1)..(3119)

<400> 3

aatgaaggtt tgggtcactc caccaggaaa aaaaaaaaca tgaactgctg aggtgcttgc 60

tgaaggcaaa gggaaatggt gaaggtgaag gaggagcaaa ggcacctctt acatggcggc 120

aggcaaagag catgtgcagg gaactgctct tcataaaacc atgagatctc atgagactta 180

ttcactctca tgagaacagc acaggaaaac cccacccccca tgattaaatt tcctcccact 240

tggtccctcc cacaacacat gaggattacg ggagctaata ttattaatac aattcaagat 300

gagatttggg tggggacaca gtcgaactgt atcaggcatc ctatgggcag gccagaagtg 360

ttggttaccc cttccaccca tgccttactg gccagaactc actcatacag ccttacctga 420

tgggggcggg gtggtggtac tgggaaatgt ggatgcacaa acgggtgtga ccacagggta 480

cagtgcttgc cacactcagc tgacgtgttt ctctcccaca caccaggcca cttggagatg 540

caagacaaag cagcccaccc ctggtcccca tcatcccctt ggaatcacct tggaaaaccc 600

tggatcattt tgaaagatgc tcatggacgc catctcattg ggctcatca cagtatcaag 660

gtagaagagg aagctgagac cagtaagatc acatggtggc tgaaggtggt gtggctagtc 720

agggacaggt cccttggcca cagagccact ggcgggtggc aatgtgccac gaccatgggg 780

tctggcagag atggcagtgt ccctggccac agtgtccagc agcagcatgg cagccgcagc 840

agggctctgt ggtcagacag gggtgctgct gtgacctgag actgtgggct cctttgaagg 900

tggtgaccct gcccctcgtg tggagcccag ctgtgctgtg ggctgagct gtggtgtcct 960

cctctttcag gatgtggctc tgttttcact gcctaccagc tctccagcct tccccttggt 1020

tcttggagac ctgatttgct tccaatcaac cccttcctac tcagtggcca gcctatttct 1080

ccatttgcaa ccagcaaccc tggctataac ttctggggaa acttctggct ataacttctg 1140

aggcacggag cggggcagga gcttgcccag ggtcacagcg ctctcggcca gtctgggagt 1200

ggcagccagg ccacctcctc ctcctgcagg aagcttcctc cacctttcag cagccctgga 1260

gccgcatgga gcagggaagg gagttgtcta ccctccggca tcctgtgtgt tccaggctgt 1320

gtgggagcag gtgcagctgc caggcatggc aggagcctgg gcccgggcca gcactcagga 1380

atgcagcagg gccctgcctc tccctgtagg gataagcaag tgccaggcgc ccagggcagc 1440

ggatgtgtct gtggctacag ccccaacggc ccccgcctcc gcaccggctg tcctgggcca 1500

gccccttggg ggcctgggat gtgtggggag catgaatggg gctctgtgac ccagcaactg 1560

ttctgcggaa ggcggctggt ggctgcacag gtgactgcgg gggtgggtgg gggatgcaaa 1620

attctgcttc ctgggcctgg tgtcccccgc cttgcatgag gccctgacaa gacatcaccg 1680

aagcaccaaa gccaaccctt gtggcccacc ggagacctgt ctcttggatc acaggggcag 1740

tggggagggg cgcccagggc tcccgatgcc tctgagccct gctctgaggt cagccgatgt 1800

ggctcagtcc tggctgtgag gcctcacgct gccctgattc catttgctcc tcagtgcgag 1860

gcagacagag cccagcctag ggtctggatg ggatgaaatg aagacgtctc ctccctaacg 1920

ggacactgtc taccccttcc ttcttctccc ccgagaaccg tcagccccgt gaggatgggg 1980

tctagggtag ggcatgtgga cggagctttg ctgtttgtcc aggtgcttat cttctagggt 2040

gccatcgccc ctccccactg ctgttcccgt atctgctggg tgtccccaac cccagggtgg 2100

tggaggccgt ctctcggatg gggctgacac ccaaggcacg gacctgccag gtcccccaaa 2160

gcacatggcc tctctgcaga agaacctgga tgtgacatct aatgcccagg ccagagcttg 2220

gggacagctg gactggagcc acagggtcaa ggagggggag tccagaagcg caaagtccac 2280

ccagctggga gggctgctgg caggtccttt acaaagcagg cagctcctct gcccatcgga 2340

gccggctggc ctaaccaggg cctgctcttg cctgggatgg gggcagaaga ggtggagacc 2400

tggggccctg aggcggcact gtgggtcctg gacccgccca cctgcactgg ggtccctgca 2460

ggcttttaat gggaacagaa atggaggaag agacagaggc tgccaggggc tccccgaccc 2520

ctactgcctg cctggggagg ggtcacctca gtgtggggga agcctgggga tgtgagagca 2580

tcctaggcct gggctgcctg tggccagtct gttgtccggg tgtctagtga cccctggggt 2640

aggggcagat gccagtctgg gaagccggat tgtttgaaga ccaactttaa agttgggagc 2700

agtgcccaga gcggggccga tgtctgctag gtggttgtct ctgctttttg aaaaagaagt 2760

ccccgcccga cccccgcccc gccaggcgct ggtctgagcg tctgagccca gatggtgcgc 2820

ttgctccaga gggcgggcgg ctccagtggc cgccgcggga cggtggggcc agaggggccc 2880

gtgggggtgg gggagccgcc cgcaggagaa ggagccccgc ccgcgccggc cctggagtcg 2940

ccggtgtcgc cgccctgccc gcgggcccgc cctcctggcc cagcccaggg ccctgcgagc 3000

tattttgaaa gtgaccctgg gctggggcgc cggggcgagc gcggcggcgc ggaaccatga 3060

cagaagatga ccgaggcggc gctggtggag ggccaggtca agctgcggga cggcaagaa 3119

<210> 4
<211> 42
<212> DNA
<213> Mus sp.

<400> 4
ggggtacctc gagccatggc cggcaacgtg aagaagagct cg 42

<210> 5
<211> 30
<212> DNA
<213> Mus sp.

<400> 5
tgccttaata ggctgccact gcctctgggg 30

<210> 6
<211> 30
<212> DNA
<213> Mus sp.

<400> 6
gtgaaatcaa aggtactgag cccatcgtgg 30

<210> 7
<211> 41
<212> DNA

<213> Mus sp.

<400> 7
gcgtcgactc gaggagagat gtgctaggtg gctacgtgag c 41

<210> 8
<211> 58
<212> DNA
<213> Artificial

<220>
<223> Description of Artificial Sequence:an artificially

synthesized primer sequence

<400> 8
agagagtagt aacaaaggtc aaagacagtt gactgtatcg atggccggca acgtgaag 58

<210> 9
<211> 60
<212> DNA
<213> Artificial

<220>
<223> Description of Artificial Sequence:an artificially

synthesized primer sequence

<400> 9
tggagacttg accaaacctc tggcgaagaa gtccaaagct ctaggtggct acgtgagcag 60

<210> 10
<211> 19
<212> DNA
<213> Homo sapiens

<400> 10
aggagggggg tttaccttg 19

<210> 11
<211> 20
<212> DNA

<213> Artificial

<220>
<223> Description of Artificial Sequence:an artificially synthesized primer sequence

<400> 11
taatacgact cactataggg 20

<210> 12
<211> 19
<212> DNA
<213> Homo sapiens

<400> 12
atgatgtcct cgtcttggc 19

<210> 13
<211> 23
<212> DNA
<213> Mus sp.

<400> 13
agcctgcgcc aggcccccag aga 23

<210> 14
<211> 24
<212> DNA
<213> Mus sp.

<400> 14
tgcatggggg ctgcctgctt ccca 24

<210> 15
<211> 20
<212> DNA
<213> Mus sp.

<400> 15
aaagtggaga ttgttgccat 20

<210> 16
<211> 19
<212> DNA
<213> Mus sp.

<400> 16
ttgactgtgc cgttgaatt 19

<210> 17
<211> 29
<212> DNA
<213> Homo sapiens

<400> 17
ttctcgagaa tgaaggtttg ggtcactcc 29

<210> 18
<211> 26
<212> DNA
<213> Homo sapiens

<400> 18
ttggatcctt cttgccgtcc cgcagc 26

<210> 19
<211> 20
<212> DNA
<213> Artificial

<220>
<223> Description of Artificial Sequence:an artificially

synthesized primer sequence

<400> 19
ttccatcttc cagcggatag 20

<210> 20
<211> 19
<212> DNA
<213> Artificial

<220>
<223> Description of Artificial Sequence:an artificially

synthesized primer sequence

<400> 20
ctaacatacg ctctccatc 19

<210> 21
<211> 21
<212> DNA
<213> Homo sapiens

<400> 21
ctcatcacag tatcaaggta g 21

<210> 22
<211> 17
<212> DNA
<213> Homo sapiens

<400> 22
gctcagacca gcgcctg 17

<210> 23
<211> 27
<212> DNA
<213> Homo sapiens

<400> 23
aagagctctg acacccaagg cacggac 27

<210> 24
<211> 27
<212> DNA
<213> Homo sapiens

<400> 24
aagagctcga tgtgagagca tcctagg 27

<210> 25
<211> 1404
<212> DNA
<213> Mus. sp

<220>
<221> CDS
<222> (1)..(1401)

<400> 25
atg ctg gtc tac aag gac aaa tgt gaa cgc tcc aag ggc ctt cgg gag 48
Met Leu Val Tyr Lys Asp Lys Cys Glu Arg Ser Lys Gly Leu Arg Glu
1 5 10 15

cgc agc agc ctc acc ctg gag gac atc tgt ggc ctg gag cct gcc ctg 96
Arg Ser Ser Leu Thr Leu Glu Asp Ile Cys Gly Leu Glu Pro Ala Leu
20 25 30

ccc tat gag ggc ctg gcc cac act ctg gcc atc atc tgc ctg tct cag 144
Pro Tyr Glu Gly Leu Ala His Thr Leu Ala Ile Ile Cys Leu Ser Gln
35 40 45

gct gtt atg ctg ggc ttc gat agc cat gag gcc atg tgt gcc tgg gat 192
Ala Val Met Leu Gly Phe Asp Ser His Glu Ala Met Cys Ala Trp Asp
50 55 60

acc cgt atc cgc tac gca ctg ggc gag gtg cac agg ttc cat gtg aca 240
Thr Arg Ile Arg Tyr Ala Leu Gly Glu Val His Arg Phe His Val Thr
65 70 75 80

gta gct cct ggt acc aaa ctg gag agt ggt cca gcc act ctt cac ctc 288
Val Ala Pro Gly Thr Lys Leu Glu Ser Gly Pro Ala Thr Leu His Leu
85 90 95

tgc aat gac att ctg gtc ctg gcc aga gac atc cct cca acc gtc atg 336
Cys Asn Asp Ile Leu Val Leu Ala Arg Asp Ile Pro Pro Thr Val Met
100 105 110

ggg cag tgg aag ctg tct gac ctc cgg cgt tac ggg gct gtt ccg aat 384
Gly Gln Trp Lys Leu Ser Asp Leu Arg Arg Tyr Gly Ala Val Pro Asn
115 120 125

gga ttc atc ttc gaa ggc ggg acc agg tgt ggg tac tgg gct gga gtc 432
Gly Phe Ile Phe Glu Gly Gly Thr Arg Cys Gly Tyr Trp Ala Gly Val
130 135 140

ttc ttc ttg tca tca gct gag gga gag cag atg agc ttc ctg ttt gac 480
Phe Phe Leu Ser Ser Ala Glu Gly Glu Gln Met Ser Phe Leu Phe Asp
145 150 155 160

tgc atc gtc cga ggc atc tcc ccg acc aaa ggc ccg ttt ggg ctt cgg 528
Cys Ile Val Arg Gly Ile Ser Pro Thr Lys Gly Pro Phe Gly Leu Arg
165 170 175

cca gtt ctc cca gac ccg agt tct ggg gga ccc tca gcc tca gaa gag 576
Pro Val Leu Pro Asp Pro Ser Ser Gly Gly Pro Ser Ala Ser Glu Glu
180 185 190

cgt gtt gcc cag gaa gca ctg gaa gcc ctg cag cta gag aag agg ctc 624
Arg Val Ala Gln Glu Ala Leu Glu Ala Leu Gln Leu Glu Lys Arg Leu
195 200 205

agc ctg ctt tct cac tct ggc cgg cca ggc agt gga ggg gat gac aga 672
Ser Leu Leu Ser His Ser Gly Arg Pro Gly Ser Gly Gly Asp Asp Arg
210 215 220

agt cta tcc agt tcc tct tct gag gct agc cac tcg gac atc agc gcc 720
Ser Leu Ser Ser Ser Ser Ser Glu Ala Ser His Ser Asp Ile Ser Ala
225 230 235 240

agc agc agg ctc act gcg tgg ccg gag cag tcc tca tcc tcg gcc ggc 768
Ser Ser Arg Leu Thr Ala Trp Pro Glu Gln Ser Ser Ser Ser Ala Gly
245 250 255

aca tca caa gaa gga cca ggg ctg gtg gct gcc cag ggc cca gga gaa 816
Thr Ser Gln Glu Gly Pro Gly Leu Val Ala Ala Gln Gly Pro Gly Glu
260 265 270

gcc atg ctg gga gcc tca agg cca ccc ctc aag cca ctg cgg cct cgg 864
Ala Met Leu Gly Ala Ser Arg Pro Pro Leu Lys Pro Leu Arg Pro Arg
275 280 285

cag tta cag gag gtt ggc cgc cag agc tcc tct gac agt ggc att gcc 912
Gln Leu Gln Glu Val Gly Arg Gln Ser Ser Ser Asp Ser Gly Ile Ala
290 295 300

aca ggc agc cac tcc tct tac tct ggc agc ttc tcc tct tat gcc ggc 960
Thr Gly Ser His Ser Ser Tyr Ser Gly Ser Phe Ser Ser Tyr Ala Gly
305 310 315 320

agc aac ctg gac gtg tgg cgg gcc ggt gag gaa ttc ggt tct ctg ctc 1008

```
Ser Asn Leu Asp Val Trp Arg Ala Gly Glu Glu Phe Gly Ser Leu Leu
                325                 330                 335

agt ctg ccc cct gga gcc agc gca cct gag ccc aga ctg tgt gcc tgc      1056
Ser Leu Pro Pro Gly Ala Ser Ala Pro Glu Pro Arg Leu Cys Ala Cys
            340                 345                 350

cca cct ggg gcg gcc gag tac cag gtg ccc acg tca ctg aga cac cac      1104
Pro Pro Gly Ala Ala Glu Tyr Gln Val Pro Thr Ser Leu Arg His His
        355                 360                 365

tat gac aca cct cga agc ctg cgc cag gcc ccc aga gac cca agc cca      1152
Tyr Asp Thr Pro Arg Ser Leu Arg Gln Ala Pro Arg Asp Pro Ser Pro
    370                 375                 380

gct tct cag ggc agc tct gac cac ggt tca gcc aca gac ttg ggt ggt      1200
Ala Ser Gln Gly Ser Ser Asp His Gly Ser Ala Thr Asp Leu Gly Gly
385                 390                 395                 400

cag gcg ccc aca ggg tgt ccc tcc agt tgg ctg gga gct cgc cga cgg      1248
Gln Ala Pro Thr Gly Cys Pro Ser Ser Trp Leu Gly Ala Arg Arg Arg
                405                 410                 415

gga cag gca acg gaa ggc cca ggc agt gac gct gcg ctg ccg agt cca      1296
Gly Gln Ala Thr Glu Gly Pro Gly Ser Asp Ala Ala Leu Pro Ser Pro
            420                 425                 430

tcc cct ggc gag tcc tgg gaa gca ggc agc ccc cat gca ggg ccg cct      1344
Ser Pro Gly Glu Ser Trp Glu Ala Gly Ser Pro His Ala Gly Pro Pro
        435                 440                 445

cca gct ttc ttt ttg tca tgt tca atc tgt ggc gga ctc aag gta aag      1392
Pro Ala Phe Phe Leu Ser Cys Ser Ile Cys Gly Gly Leu Lys Val Lys
    450                 455                 460

ccc cct ccc tga                                                      1404
Pro Pro Pro
465
```

<210> 26
<211> 467
<212> PRT
<213> Mus. sp

<400> 26

Met Leu Val Tyr Lys Asp Lys Cys Glu Arg Ser Lys Gly Leu Arg Glu
1               5                   10                  15

Arg Ser Ser Leu Thr Leu Glu Asp Ile Cys Gly Leu Glu Pro Ala Leu
            20                  25                  30

Pro Tyr Glu Gly Leu Ala His Thr Leu Ala Ile Ile Cys Leu Ser Gln
        35                  40                  45

Ala Val Met Leu Gly Phe Asp Ser His Glu Ala Met Cys Ala Trp Asp
    50                  55                  60

Thr Arg Ile Arg Tyr Ala Leu Gly Glu Val His Arg Phe His Val Thr
65                  70                  75                  80

Val Ala Pro Gly Thr Lys Leu Glu Ser Gly Pro Ala Thr Leu His Leu
                85                  90                  95

Cys Asn Asp Ile Leu Val Leu Ala Arg Asp Ile Pro Pro Thr Val Met
            100                 105                 110

Gly Gln Trp Lys Leu Ser Asp Leu Arg Arg Tyr Gly Ala Val Pro Asn
        115                 120                 125

Gly Phe Ile Phe Glu Gly Gly Thr Arg Cys Gly Tyr Trp Ala Gly Val
    130                 135                 140

Phe Phe Leu Ser Ser Ala Glu Gly Glu Gln Met Ser Phe Leu Phe Asp
145                 150                 155                 160

Cys Ile Val Arg Gly Ile Ser Pro Thr Lys Gly Pro Phe Gly Leu Arg
                165                 170                 175

Pro Val Leu Pro Asp Pro Ser Ser Gly Gly Pro Ser Ala Ser Glu Glu

180 185 190

Arg Val Ala Gln Glu Ala Leu Glu Ala Leu Gln Leu Glu Lys Arg Leu
195 200 205

Ser Leu Leu Ser His Ser Gly Arg Pro Gly Ser Gly Gly Asp Asp Arg
210 215 220

Ser Leu Ser Ser Ser Ser Ser Glu Ala Ser His Ser Asp Ile Ser Ala
225 230 235 240

Ser Ser Arg Leu Thr Ala Trp Pro Glu Gln Ser Ser Ser Ser Ala Gly
245 250 255

Thr Ser Gln Glu Gly Pro Gly Leu Val Ala Ala Gln Gly Pro Gly Glu
260 265 270

Ala Met Leu Gly Ala Ser Arg Pro Pro Leu Lys Pro Leu Arg Pro Arg
275 280 285

Gln Leu Gln Glu Val Gly Arg Gln Ser Ser Ser Asp Ser Gly Ile Ala
290 295 300

Thr Gly Ser His Ser Ser Tyr Ser Gly Ser Phe Ser Ser Tyr Ala Gly
305 310 315 320

Ser Asn Leu Asp Val Trp Arg Ala Gly Glu Glu Phe Gly Ser Leu Leu
325 330 335

Ser Leu Pro Pro Gly Ala Ser Ala Pro Glu Pro Arg Leu Cys Ala Cys
340 345 350

Pro Pro Gly Ala Ala Glu Tyr Gln Val Pro Thr Ser Leu Arg His His
355 360 365

Tyr Asp Thr Pro Arg Ser Leu Arg Gln Ala Pro Arg Asp Pro Ser Pro
    370                 375                 380

Ala Ser Gln Gly Ser Ser Asp His Gly Ser Ala Thr Asp Leu Gly Gly
385                 390                 395                 400

Gln Ala Pro Thr Gly Cys Pro Ser Ser Trp Leu Gly Ala Arg Arg Arg
                405                 410                 415

Gly Gln Ala Thr Glu Gly Pro Gly Ser Asp Ala Ala Leu Pro Ser Pro
            420                 425                 430

Ser Pro Gly Glu Ser Trp Glu Ala Gly Ser Pro His Ala Gly Pro Pro
        435                 440                 445

Pro Ala Phe Phe Leu Ser Cys Ser Ile Cys Gly Gly Leu Lys Val Lys
    450                 455                 460

Pro Pro Pro
465

<210> 27
<211> 23
<212> DNA
<213> Mus. sp

<400> 27
atgctggtct acaaggacaa atg 23

<210> 28
<211> 21
<212> DNA
<213> Mus. sp

<400> 28
ccagcctgct atctcaggga g 21

<210> 29
<211> 21
<212> DNA
<213> Mus. sp

<400> 29
gggagggggc tttaccttga g 21

<210> 30
<211> 25
<212> DNA
<213> Artificial

<220>
<223> Description of Artificial Sequence:an artificially synthesized primer sequence

<400> 30
aagatatcca tggccggcaa cgtgg 25

<210> 31
<211> 25
<212> DNA
<213> Artificial

<220>
<223> Description of Artificial Sequence:an artificially synthesized primer sequence

<400> 31
aactcgagta cgtgagcagg agaag 25

<210> 32
<211> 19
<212> DNA
<213> Mus. sp

<400> 32
tttgcagggc tggcaagcc 19

<210> 33

<211> 27
<212> DNA
<213> Artificial

<220>
<223> Description of Artificial Sequence:an artificially synthesized primer sequence

<400> 33
ttggatccat gatgtcctcg tcttggc 27

<210> 34
<211> 18
<212> DNA
<213> Artificial

<220>
<223> Description of Artificial Sequence:an artificially synthesized primer sequence

<400> 34
tagaaggcac agtcgagg 18